(19) Europäisches Patentamt
European Patent Office
Office européen des brevets

(11) EP 4 610 227 A1

(12) # EUROPEAN PATENT APPLICATION
published in accordance with Art. 153(4) EPC

(43) Date of publication:
**03.09.2025 Bulletin 2025/36**

(21) Application number: **23881948.6**

(22) Date of filing: **27.10.2023**

(51) International Patent Classification (IPC):
***C01B 39/08*** *(2006.01)* ***B01J 29/89*** *(2006.01)*
***C07C 249/04*** *(2006.01)*

(52) Cooperative Patent Classification (CPC):
**B01J 29/89; C01B 37/00; C01B 39/08; C07C 249/04; C07D 201/04; C07D 223/10;** Y02P 20/52

(86) International application number:
**PCT/CN2023/127042**

(87) International publication number:
**WO 2024/088373 (02.05.2024 Gazette 2024/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.10.2022 CN 202211328795**

(71) Applicants:
- **China Petroleum & Chemical Corporation Beijing 100728 (CN)**
- **Sinopec Research Institute of Petroleum Processing Co., Ltd. Beijing 100083 (CN)**

(72) Inventors:
- **XIA, Changjiu Beijing 100083 (CN)**
- **ZHANG, Peng Beijing 100083 (CN)**
- **PENG, Xinxin Beijing 100083 (CN)**
- **XING, Enhui Beijing 100083 (CN)**
- **ZHANG, Xiaoxin Beijing 100083 (CN)**
- **LUO, Yibin Beijing 100083 (CN)**
- **LIN, Min Beijing 100083 (CN)**
- **ZHU, Bin Beijing 100083 (CN)**
- **SHU, Xingtian Beijing 100083 (CN)**

(74) Representative: **Hoffmann Eitle Patent- und Rechtsanwälte PartmbB Arabellastraße 30 81925 München (DE)**

(54) **TITANIUM SILICALITE MOLECULAR SIEVE HAVING HIERARCHICAL-PORE STRUCTURE AND PREPARATION THEREFOR AND USE THEREOF**

(57) Disclosed is a titanium silicalite molecular sieve with a hierarchical porous structure, wherein the spectral peaks of the $^1H$ MAS NMR spectrum of the titanium silicalite molecular sieve in the chemical shift range of 1-6 ppm are separated to obtain five spectral peaks whose chemical shifts corresponding to the peak positions are 1.8±0.1 ppm, 2.2 ppm±0.1 ppm, 2.8 ppm+0.1 ppm, 3.8±0.1 ppm and 4.6±0.1 ppm, respectively, and the peak areas of the peaks satisfy specific proportional relationships. The molecular sieve has significantly enhanced anti-deactivation performance and can significantly improve the cyclohexanone oxime conversion rate and the caprolactam selectivity when used in the cyclohexanone oxime gas-phase Beckmann rearrangement reaction.

EP 4 610 227 A1

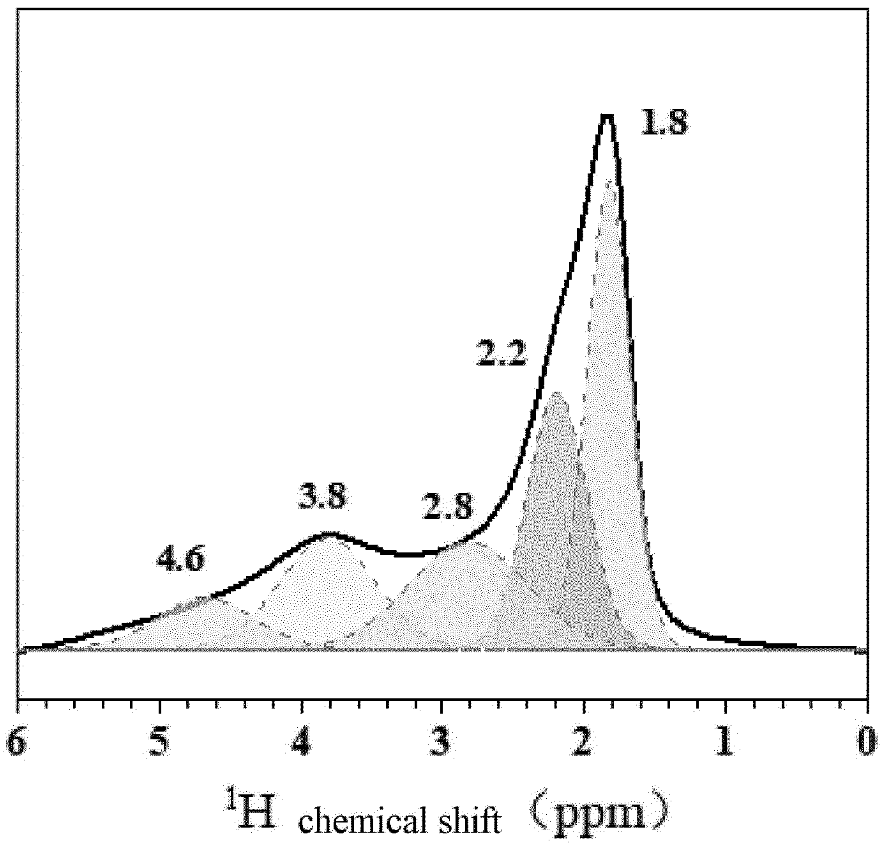


Fig. 1

## Description

### Technical Field

The present application relates to the technical field of titanium silicalite molecular sieves, and in particular to a titanium silicalite molecular sieve with a hierarchical porous structure and its preparation and application.

### Background Art

Beckmann rearrangement reaction is a process that converts ketoxime compounds into amides. Its most important application in chemical industry production is the production of caprolactam. Caprolactam is an important monomer for the synthesis of nylon-6. It is widely used in the production of important downstream products such as engineering plastics, nylon-6 fibers and industrial cord fabrics. It can also be used in the fields of coatings, pharmaceuticals and fine chemicals.

At present, more than 95% of caprolactam production adopts the cyclohexanone oxime liquid-phase Beckmann rearrangement reaction process, in which concentrated sulfuric acid (or fuming sulfuric acid) is used as a catalyst and solvent, which has serious problems such as equipment corrosion and environmental pollution. After the reaction, liquid ammonia is used to neutralize the waste sulfuric acid, producing a large amount of low-value ammonium sulfate by-product (1.9t ammonium sulfate/t caprolactam), resulting in poor technical and economic performance of this route.

Therefore, the cyclohexanone oxime gas-phase Beckmann rearrangement process based on molecular sieve catalysts has received great attention from academia and industry. Compared with the traditional liquid phase method, this process avoids the use of ammonia and fuming sulfuric acid from the source, and the atomic utilization rate is close to 100%, which is an environmentally friendly caprolactam green production process. Studies have found that in the cyclohexanone oxime gas-phase Beckmann rearrangement reaction, all-silica molecular sieves with MFI topological structure show excellent catalytic performance. Therefore, at the beginning of this century, Japan Sumitomo and Sinopec have successively used all-silica molecular sieve to carry out industrial experiments of the cyclohexanone oxime gas-phase Beckmann rearrangement.

However, the gas-phase Beckmann rearrangement route has two problems: poor product CPL selectivity and short catalyst single-pass life, which affect the technical economy and operational continuity and stability, resulting in extremely slow subsequent large-scale industrial expansion and commercial promotion. This is because the micropore size of the MFI structure is small, close to the molecular size of the reactants and products, which leads to slow diffusion of guest molecules in the confined pores of the molecular sieve and significantly prolonged residence time in the crystal, thus aggravating the generation of by-products and the clogging of the pores by carbon deposits. In addition, since the silanol group active center of the all-silica molecular sieve is unstable, it can be easily deactivated by heat and alkaline byproducts. It is necessary to develop processes to further enhance the catalyst's resistance to deactivation.

To this end, Baojun Li's research group (RSC Adv., 2013, 3, 20811-20815) added S-1 and TS-1 molecular sieves as seeds during the crystallization of molecular sieves, and synthesized titanium silicalite molecular sieves with different particle sizes for the gas-phase Beckmann rearrangement reaction of cyclohexanone oxime. Ferdi Schüth et al. (Microporous and Mesoporous Materials, 2009, 117, 228-232) added 1,7-dichlorooctamethyltetraoxysilane to form pores during the synthesis of titanium silicalite molecular sieves, and obtained hierarchical porous titanium silicalite molecular sieves, which were used for the gas-phase Beckmann rearrangement reaction of cyclohexanone oxime.

However, when titanium silicalite molecular sieve synthesized by existing processes is used in the gas-phase Beckmann rearrangement reaction of cyclohexanone oxime, the effect of improving caprolactam selectivity and catalyst life is not ideal, and there is still a certain gap from the requirements of industrial production.

### Summary of the invention

The purpose of the present application is to provide a titanium silicalite molecular sieve with a hierarchical porous structure and its preparation and application, wherein the molecular sieve has significantly enhanced anti-deactivation performance and can significantly improve the cyclohexanone oxime conversion rate and caprolactam selectivity when used in the cyclohexanone oxime gas-phase Beckmann rearrangement reaction.

In order to achieve the above object, in one aspect, the present application provides a titanium silicalite molecular sieve with a hierarchical porous structure, wherein the spectral peaks of the $^{1}H$ MAS NMR spectrum of the titanium silicalite molecular sieve in the chemical shift range of 1-6 ppm are separated to obtain five spectral peaks whose chemical shifts corresponding to the peak positions are 1.8±0.1 ppm, 2.2 ppm±0.1 ppm, 2.8 ppm±0.1 ppm, 3.8±0.1 ppm and 4.6±0.1 ppm, respectively, wherein:

the peak area of the spectral peak whose peak is at 1.8±0.1 ppm is recorded as $A_1$, the peak area of the spectral peak whose peak is at 2.2 ppm±0.1 ppm is recorded as $A_2$, the peak area of the spectral peak whose peak is at 2.8 ppm±0.1

ppm is recorded as $A_3$, the peak area of the spectral peak whose peak is at 3.8±0.1 ppm is recorded as $A_4$, and the peak area of the spectral peak whose peak is at 4.6±0.1 ppm is recorded as $A_5$, then:

$X_1$ as defined by the following formula (1) is in the range of 0.5-2.2:

$$X_1 = (A_2 + A_3)/A_1 \qquad (1);$$

$X_2$ as defined by the following formula (2) is in the range of 0. 1-1.2:

$$X_2 = A_4/A_1 \qquad (2);$$

$X_3$ as defined by the following formula (3) is in the range of 0.05-0.65:

$$X_3 = A_5/A_1 \qquad (3).$$

In another aspect, the present application provides a method for preparing the titanium silicalite molecular sieve with a hierarchical porous structure of the present application, comprising the following steps:

1) mixing a titanium source, a silicon source, a first templating agent, water, a hard templating agent and a high molecular weight polymer to obtain a reaction mixture, wherein the hard templating agent is selected from one or more of a carbon material, a resin material and a solid inorganic compound;
2) sequentially performing a first hydrothermal crystallization treatment and a first calcination treatment on the reaction mixture to obtain a molecular sieve intermediate;
3) mixing the molecular sieve intermediate, a second templating agent and water, and then sequentially performing a second hydrothermal crystallization treatment and a second calcination treatment.

Preferably, the first templating agent and the second templating agent are organic bases, and are independently selected from quaternary ammonium bases, aliphatic amines, aliphatic alcohol amines, or a combination thereof.
In another aspect, the present application provides a method for preparing caprolactam from cyclohexanone oxime, comprising: subjecting cyclohexanone oxime to a gas-phase Beckmann rearrangement reaction in the presence of the titanium silicalite molecular sieve with a hierarchical porous structure of the present application.
The titanium silicalite molecular sieve of the present application introduces a novel of titanium atom active center into the molecular sieve framework, which effectively improves the anti-deactivation performance of the molecular sieve and helps to extend the service life of the molecular sieve under alkaline reaction conditions; at the same time, its hierarchical porous structure gives the molecular sieve abundant silanol groups with hydrogen bond interactions, so that $X_1$, $X_2$ and $X_3$ defined in formulas (1) to (3) are within a specific numerical range, thereby enabling the titanium silicalite molecular sieve to significantly improve the cyclohexanone oxime conversion rate and caprolactam selectivity when used in the gas-phase Beckmann rearrangement reaction of cyclohexanone oxime; in addition, the hierarchical porous structure of the titanium silicalite molecular sieve also gives it advantages such as large specific surface area and pore volume, which is conducive to the catalytic reaction.
Other features and advantages of the present application will be described in detail in the subsequent Specific Mode for Carrying out the Invention section.

## Description of Drawings

The drawings are used to provide a further understanding of the present application and constitute a part of the specification. Together with the following specific embodiments, they are used to explain the present application but do not constitute a limitation to the present application. In the drawings:

FIG 1 is a $^1H$ MAS NMR spectrum of the molecular sieve obtained in Example 1;
FIG2 is a $^{31}P$ MAS NMR spectrum of the molecular sieve obtained in Example 1;
FIG3 is an XRD spectrum of the molecular sieve obtained in Example 1;
FIG4 is a TEM electron microscope image of the molecular sieve obtained in Example 1;
FIG5 is a SEM electron microscope image of the molecular sieve obtained in Example 1;
FIG6 is a BET curve diagram of the molecular sieve obtained in Example 1;
FIG7 is a TEM electron microscope image of the intermediate product obtained in Example 1;

FIG8 is a $^1H$ MAS NMR spectrum of the intermediate product obtained in Example 1;
FIG9 is a $^{31}P$ MAS NMR spectrum of the intermediate product obtained in Example 1;
FIG10 is a TEM electron microscope image of the molecular sieve obtained in Comparative Example 2;
FIG11 is a $^1H$ MAS NMR spectrum of the molecular sieve obtained in Comparative Example 2;
FIG12 is a $^{31}P$ MAS NMR spectrum of the molecular sieve obtained in Comparative Example 2;
FIG13 is an XRD spectrum of the molecular sieve product obtained in Example 10;
FIG. 14 is an XRD spectrum of the molecular sieve product obtained in Example 11.

## Specific Mode for Carrying out the Invention

The specific implementation of the present application is described in detail below. It should be understood that the specific implementation described here is only used to illustrate and explain the present application, and is not used to limit the present application.

Any specific numerical value disclosed herein (including the endpoint of the numerical range) is not limited to the exact value of the numerical value, but should be understood to also cover values close to the exact value, such as all possible values within the range of $\pm5\%$ of the exact value. In addition, for the disclosed numerical range, the endpoint values of the range, the endpoint values and the specific point values in the range, and the specific point values can be arbitrarily combined to obtain one or more new numerical ranges, and these new numerical ranges should also be regarded as specifically disclosed herein.

Unless otherwise specified, the terms used herein have the same meaning as commonly understood by those skilled in the art. If a term is defined herein and its definition is different from the commonly understood meaning in the art, the definition herein shall prevail.

In the present application, the term "hierarchical porous structure" has the meaning generally understood in the art, specifically referring to a molecular sieve having multiple levels of pore sizes, for example, two levels of pore sizes of micropores (pore size <2 nm) and mesopores (pore size of 2-50 nm), micropores and macropores (pore size > 50 nm), or mesopores and macropores, or three levels of pore sizes of micropores, mesopores and **macropores** (pore size > 50 nm).

In the present application, the cavity structure in the molecular sieve crystal grains and its size and volume are obtained by transmission electron microscopy (TEM) testing, wherein the size of a single cavity structure refers to the maximum length of the line between two positions where the lines on the cavity wall of the cavity structure pass through the center of the cavity structure in the transmission electron microscope image of the molecular sieve, and the average value of the size of all the cavity structures in the crystal grain is calculated as the size of the cavity structure of the crystal grain, and the average value of the size of the cavity structure of 50 molecular sieve crystal grains is taken as the size of the cavity structure of the molecular sieve. For example, "the size of the cavity structure is within the range of 5-50nm" means that the size of the molecular sieve cavity structure represented by the above average value is within the range of 5-50nm; preferably, the length of the line between any two positions on the cavity wall of any cavity structure in each molecular sieve crystal grain passing through the center of the cavity structure is within the range of 5-50nm. The volume of the cavity structure is estimated according to the sphere, wherein the arithmetic mean of the sum of the lengths of the longest line and the shortest line among all lines between two positions on the cavity wall passing through the center of the cavity structure is taken as the spherical diameter, and then the spherical radius is obtained, and then the volume of the corresponding cavity structure is calculated according to the spherical volume formula. The percentage of the sum of the volumes of all the cavity structures in the molecular sieve crystal grains to the volume of the corresponding molecular sieve crystal grains is calculated, and the average value of the volume fractions of the cavity structures of 50 molecular sieve crystal grains is taken as the percentage of the cavity structure volume of the molecular sieve to the volume of the molecular sieve.

In the present application, the peak intensity of each characteristic peak in the $^{31}P$ MAS NMR spectrum of the molecular sieve is represented by the integrated area of the corresponding characteristic peak.

**In the** present application, except for the contents clearly stated, any matters or items not mentioned are directly applicable to those known in the art without any changes. Moreover, any embodiment described herein can be freely combined with one or more other embodiments described herein, and the technical solutions or technical ideas formed thereby are deemed to be part of the original disclosure or original record of the present application, and should not be regarded as new contents not disclosed or anticipated herein, unless a person skilled in the art considers that the combination is obviously unreasonable.

All patent and non-patent literatures, including but not limited to textbooks and journal articles, mentioned herein are incorporated herein by reference in their entirety.

The inventor of the present application has found through a large number of experimental studies that in the $^1H$ MAS NMR spectrum of the titanium silicalite molecular sieve, there are the following two silanol group active centers: an independent silanol group active center with a chemical shift at a position of $1.8 \pm 0.1$ ppm, and a silanol group active center with hydrogen bond interaction with a chemical shift in the range of 2-6 ppm. By separating the peaks, it is found that the peak of the silanol group active center with hydrogen bond interaction includes four peaks with chemical shifts at

positions of 2.2 ± 0.1 ppm, 2.8 ± 0.1 ppm, 3.8 ± 0.1 ppm and 4.6 ± 0.1 ppm, respectively. When the value of the ratios $X_1$ -$X_3$ between the peak areas of the peaks of different types of silanol group active centers are within a specific range, the obtained titanium silicalite molecular sieve has good catalytic activity and catalytic stability, especially when used in the gas-phase Beckmann rearrangement reaction of cyclohexanone oxime, the cyclohexanone oxime conversion rate and caprolactam selectivity can be effectively improved, thereby obtaining the present invention.

As described above, in a first aspect, the present application provides a titanium silicalite molecular sieve having a hierarchical porous structure, wherein the spectral peaks of the $^1$H MAS NMR spectrum of the titanium silicalite molecular sieve in the chemical shift range of 1-6 ppm are separated to obtain five spectral peaks whose chemical shifts corresponding to the peak positions are 1.8±0.1 ppm, 2.2 ppm±0.1 ppm, 2.8 ppm±0.1 ppm, 3.8±0.1 ppm and 4.6±0.1 ppm, respectively, wherein:

the peak area of the spectral peak whose peak is at 1.8±0.1 ppm is recorded as $A_1$, the peak area of the spectral peak whose peak is at 2.2 ppm±0.1 ppm is recorded as $A_2$, the peak area of the spectral peak whose peak is at 2.8 ppm±0.1 ppm is recorded as $A_3$, the peak area of the spectral peak whose peak is at 3.8±0.1 ppm is recorded as $A_4$, and the peak area of the spectral peak whose peak is at 4.6±0.1 ppm is recorded as $A_5$, then:

$X_1$ as defined by the following formula (1) is in the range of 0.5-2.2:

$$X_1 = (A_2 + A_3)/A_1 \qquad (1);$$

$X_2$ as defined by the following formula (2) is in the range of 0. 1-1.2:

$$X_2 = A_4/A_1 \qquad (2);$$

$X_3$ as defined by the following formula (3) is in the range of 0.05-0.65:

$$X_3 = A_5/A_1 \qquad (3).$$

Without being limited to a specific theory, it is believed that the titanium silicalite molecular sieve of the present application introduces a novel hexacoordinated titanium atom active center into the molecular sieve framework, which effectively improves the anti-deactivation performance of the molecular sieve and helps to extend the service life of the molecular sieve under alkaline reaction conditions; at the same time, its hierarchical porous structure gives the molecular sieve abundant silanol groups with hydrogen bond interactions, so that $X_1$, $X_2$ and $X_3$ defined in formulas (1) to (3) are within a specific numerical range, thereby enabling the titanium silicalite molecular sieve to significantly improve the cyclohexanone oxime conversion rate and caprolactam selectivity when used in the gas-phase Beckmann rearrangement reaction of cyclohexanone oxime; in addition, the hierarchical porous structure of the titanium silicalite molecular sieve also gives it advantages such as large specific surface area and pore volume, which is conducive to the catalytic reaction.

In the present application, $^1$H MAS NMR testing can be performed by using conventional testing instruments and testing methods in the art, and conventional processing software and method can be used to perform peak separation, integration, and other processing on the spectral peaks.

In a preferred embodiment, the value of $X_1$ is in the range of 0.6-1.8; the value of $X_2$ is in the range of 0.15-1.10; and the value of $X_3$ is in the range of 0.10-0.55. When the values of $X_1$-$X_3$ of the titanium silicalite molecular sieve are in the above preferred range, the titanium silicalite molecular sieve shows higher cyclohexanone oxime conversion rate and caprolactam selectivity in the gas-phase Beckmann rearrangement reaction of cyclohexanone oxime, and the catalytic stability of the molecular sieve is better under long-term reaction conditions.

In a preferred embodiment, the solid nuclear magnetic resonance characterization is performed using trimethylphosphine (TMP) as a probe molecule, and the $^{31}$P MAS NMR spectrum of the molecular sieve shows three characteristic peaks at chemical shifts of -5±1 ppm, -34±1 ppm and - 61±1 ppm, respectively, wherein the peak intensity of the characteristic peak at the chemical shift of -5±1 ppm is recorded as $N_1$, the peak intensity of the characteristic peak at the chemical shift of -34±1 ppm is recorded as $N_2$, and the peak intensity of the characteristic peak at the chemical shift of -61 ±1 ppm is recorded as $N_3$, then:

$Y_1$ as defined by the following formula (4) is in the range of 0.005-0.15, preferably in the range of 0.02-0.12:

$$Y_1 = N_1/N_3 \qquad (4);$$

and

$Y_2$ as defined by the following formula (5) is in the range of 0.10-0.30, preferably in the range of 0. 12-0.26:

$$Y_2 = N_2 / N_3 \quad (5).$$

After extensive research, the inventors of the present application also found that when trimethylphosphine (TMP) is used as a probe molecule to characterize the titanium silicalite molecular sieve by solid nuclear magnetic resonance technology, in the resulting $^{31}P$ MAS NMR spectrum, a characteristic peak characterizing the Brönsted acid property appears at a chemical shift of -5±1 ppm, a characteristic peak characterizing the Lewis acid property appears at a chemical shift of -34±1 ppm, and a characteristic peak characterizing the physical adsorption of TMP appears at a chemical shift of -61±1 ppm. Taking the characteristic peak of TMP physical adsorption as a comparison baseline, the characteristic peak characterizing the Brönsted acid property and the characteristic peak characterizing the Lewis acid property are respectively compared with the peak intensity of the characteristic peak of the TMP physical adsorption. The ratio of the peak intensity of each of the characteristic peaks characterizing the two acid properties of the titanium silicalite molecular sieve of the present application to the peak intensity of the characteristic peak of the TMP physical adsorption is within a specific range, thereby making the molecular sieve of the present application have suitable acidity and a synergistic catalytic effect on the gas-phase Beckmann rearrangement reaction of cyclohexanone oxime, thereby further improving the caprolactam selectivity and extending the catalyst life.

In the present application, trimethylphosphine (TMP) is used as a probe molecule in the $^{31}P$ MAS NMR spectrum test of the titanium silicalite molecular sieve, and the test method is a conventional method in the art.

In a preferred embodiment, the molar ratio of silicon atom to titanium atom in the titanium silicalite molecular sieve of the present application is (8-150): 1, preferably (15-120): 1. In the present application, the molar ratio of silicon atom to titanium atom in the molecular sieve can be obtained by X-ray fluorescence spectroscopy analysis.

In a preferred embodiment, the titanium silicalite molecular sieve of the present application has a plurality of cavity structures in the crystal grains; and the size of the cavity structure is in the range of 5-50 nm, preferably in the range of 7-45 nm. In such preferred embodiments, the cavity structure is connected to the outside through micropores, and the cavity structure and the micropores connected thereto form a hierarchical porous structure.

In such preferred embodiments, the titanium silicalite molecular sieve of the present application has a plurality of relatively large-sized cavity structures inside, thereby having a large specific surface area and pore volume. At the same time, the cavity structure has abundant silanol group active centers, thereby providing a large number of independent reaction units, and the multi-cavity structure forms more defect sites, thereby generating more silanol groups, which in turn result in stronger hydrogen bonding interactions between each other; in addition, the relatively large-sized cavity structure meets the needs of macromolecular reactions, and the reaction products are easier to flow out of the catalyst, avoiding the phenomenon of reduced catalytic activity of the catalyst due to pore blockage.

In a further preferred embodiment, the cavity structure volume of the molecular sieve accounts for 8-55 % of the volume of the molecular sieve, further preferably 12-50%, and further more preferably 20-50%. The shape of the cavity structure is not strictly limited, and can be, for example, one or more of a sphere, an ellipsoid, a cylinder, a polyhedron (such as a cube and an irregular cube), and an irregular shape, usually an irregular shape.

In some specific embodiments, the titanium silicalite molecular sieve includes molecular sieve particles composed of a single crystal grain, and/or molecular sieve particles composed of a plurality of crystal grains aggregated. Optionally, the average particle size of the molecular sieve particles is 0.1-0.7 μ m, preferably 0.13-0.62 μm; the BET specific surface area is 260-620 $m^2/g$, preferably 280-540 $m^2/g$; the micropore specific surface area is 280-500 $m^2/g$, preferably 300-480 $m^2/g$; the total pore volume is 0.16-0.55 $cm^3/g$, preferably 0.22-0.45 $cm^3/g$; the mesopore volume is 0.09-0.38 $cm^3/g$, preferably 0.12-0.34 $cm^3/g$.

In a preferred embodiment, a hysteresis loop exists between the adsorption isotherm and the desorption isotherm of low temperature nitrogen adsorption of the titanium silicalite molecular sieve. Further preferably, the initial relative pressure ($P/P_0$) at which the hysteresis loop appears is in the range of 0.35-0.55, preferably in the range of 0.38-0.48.

In a preferred embodiment, the configuration of the titanium silicalite molecular sieve is selected from the group consisting of MFI topological structure, MEL topological structure, BEA topological structure and SVR topological structure; and the MFI topological structure is further preferred.

In a second aspect, the present application provides a method for preparing a titanium silicalite molecular sieve having a hierarchical porous structure, in particular the titanium silicalite molecular sieve of the present application, comprising the following steps:

1) mixing a titanium source, a silicon source, a first templating agent, water, a hard templating agent and a high molecular weight polymer to obtain a reaction mixture, wherein the hard templating agent is selected from one or more

of a carbon material, a resin material and a solid inorganic compound;
2) sequentially performing a first hydrothermal crystallization treatment and a first calcination treatment on the reaction mixture to obtain a molecular sieve intermediate;
3) mixing the molecular sieve intermediate, a second templating agent and water, and then sequentially performing a second hydrothermal crystallization treatment and a second calcination treatment.

In the method of the present application, the space filling effect of the hard templating agent and the hydrogen bonding effect between the high molecular weight polymer and the silanol group of the organic silicon source are combined to ensure the realization of the abundant open channels and the pore expansion effect. Then, the templating agent is introduced into the molecular sieve with open channels, and the titanium silicalite molecular sieve with a hierarchical porous structure is obtained by dissolution and recrystallization mechanism of the templating agent.

Specifically, in the method of the present application, the titanium source is introduced into the molecular sieve synthesis raw material, which can introduce titanium atomic active centers into the molecular sieve framework during the molecular sieve synthesis to improve the performance of the molecular sieve; at the same time, the hard templating agent and the high molecular weight polymer are further introduced into the molecular sieve synthesis raw material, and the pores between the hard templating agent and the inorganic molecular sieve framework are expanded mainly through space filling, and the high molecular weight polymer helps to improve the problem that the hierarchical porous structure synthesized by the hard templating agent method is a closed isolated space that is not interconnected. It self-assembles with inorganic aluminosilicates through hydrogen bonds or electrostatic attraction, so that after the hard templating agent and the high molecular weight polymer are removed by the first calcination treatment, the molecular sieve intermediate with abundant open channels can be formed. Then, in step 3), the second templating agent is introduced into the molecular sieve intermediate with open channels, and its dissolution and recrystallization mechanism is utilized to obtain the titanium silicalite molecular sieve with a specific distribution of silanol group active centers and a hierarchical porous structure of the present application. Specifically, in step 3), the inside of the crystal grains of the molecular sieve intermediate obtained in step 2) is dissolved in the presence of the second templating agent in a hydrothermal environment to produce a cavity structure with a size ranging from 5 to 50 nm, and recrystallization occurs on the surface of the crystal grains, changing the crystal grain morphology; at the same time, due to the generation of this multi-cavity structure, the curvature of the silanol group distribution becomes larger, causing the silanol group density and orientation to change, increasing the hydrogen bonding effect, enhancing the hydrogen bonding effect, and increasing the adsorption force on the reaction molecules, thereby further improving the reaction performance.

In a preferred embodiment, in step 1), the molar ratio of the titanium source: the silicon source: the first templating agent: water is (0.005-0.55): 1: (0.02-1.8): (1-50); the silicon source is calculated in the form of $SiO_2$, and the weight ratio of the hard templating agent: the high molecular weight polymer: the silicon source is (0.02-0.75): (0.01-0.55): 1.

In a further preferred embodiment, in step 1), the molar ratio of the titanium source: the silicon source: the first templating agent: water is (0.01-0.42): 1: (0.04-1.5): (10-45); the silicon source is calculated in the form of $SiO_2$, and the weight ratio of the hard templating agent: the high molecular weight polymer: the silicon source is (0.04-0.55): (0.03-0.4): 1. The titanium silicalite molecular sieve prepared in this preferred embodiment has higher catalytic activity and catalytic stability.

According to the present application, the silicon source described in step 1) is not strictly limited, and can be various silicon sources suitable for the preparation of titanium silicalite molecular sieve. In a preferred embodiment, in step 1), the silicon source is selected from organic silicate, solid silica gel, white carbon black and silica sol, or a combination thereof; preferably selected from organic silicate, solid silica gel and white carbon black, or a combination thereof.

In a further preferred embodiment, the silicon source is selected from at least one of the organic silicates having the structure shown in the following formula (A):

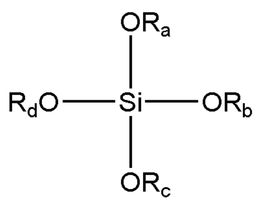

(A);

wherein $R_a$, $R_b$, $R_c$ and $R_d$ are each independently selected from linear or branched alkyl group having 1 to 6 carbon atoms, preferably $R_a$, $R_b$, $R_c$ and $R_d$ are each independently selected from linear alkyl group having 1 to 4 carbon atoms or branched alkyl group having 3 to 4 carbon atoms, and further preferably $R_a$, $R_b$, $R_c$ and $R_d$ are each independently selected from methyl, ethyl, n-propyl, isopropyl, n-butyl, sec-butyl, isobutyl or tert-butyl.

In a particularly preferred embodiment, the organic silicate is selected from tetramethyl silicate, tetraethyl silicate,

tetrabutyl silicate, dimethyl diethyl silicate, or a combination thereof.

According to the present application, the first templating agent in step 1) and the second templating agent in step 3) can be independently selected from various templating agents suitable for the preparation of titanium silicalite molecular sieves, and the present application has no strict restrictions. In a preferred embodiment, the first templating agent and the second templating agent are both organic bases, preferably independently selected from quaternary ammonium bases, aliphatic amines, aliphatic alcohol amines, or a combination thereof.

In a further preferred embodiment, the first templating agent and the second templating agent are each independently selected from at least one of the quaternary ammonium base having a structure represented by the following formula (B):

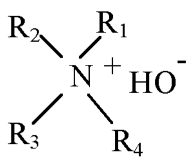
(B);

wherein $R_1$, $R_2$, $R_3$ and $R_4$ are each independently selected from alkyl group having 1 to 4 carbon atoms, preferably selected from linear alkyl group having 1 to 4 carbon atoms and branched alkyl group having 3 to 4 carbon atoms, and further preferably $R_1$, $R_2$, $R_3$ and $R_4$ are each independently selected from methyl, ethyl, n-propyl, isopropyl, n-butyl, sec-butyl, isobutyl and tert-butyl.

In a particularly preferred embodiment, the first templating agent and the second templating agent are each independently selected from tetraethylammonium hydroxide, tetrapropylammonium hydroxide, tetrabutylammonium hydroxide, tetrapropylammonium chloride, tetrapropylammonium bromide, or a combination thereof.

In a preferred embodiment, step 1) further comprises:

a) mixing the titanium source, the silicon source, the first templating agent and water to obtain a hydrolyzed sol containing titanium and silicon;
b) adding the hard templating agent and the high molecular weight polymer to the hydrolyzed sol containing titanium and silicon respectively, and mixing them to obtain the reaction mixture;

optionally, the mixing conditions in step a) include: stirring at 40-90°C for 6-12 h; and
optionally, the mixing conditions in step b) include: stirring at 20-50°C for 2-4 h.

In certain further preferred embodiments, the silicon source in step a) is an organic silicate, and step a) further comprises a hydrolysis and alcohol removal treatment after mixing the titanium source, the silicon source, the first templating agent and water to obtain the hydrolyzed sol containing titanium and silicon; optionally, the conditions of the hydrolysis and alcohol removal treatment include: stirring and hydrolyzing at 40-90 °C for 6-12 h; preferably stirring and hydrolyzing at 60-85 °C for 8-10 h. Preferably, the hydrolysis and alcohol removal treatment is performed so that the mass content of the alcohol produced by hydrolysis of the organic silicate in the hydrolyzed sol containing titanium and silicon is 10 ppm or less.

According to the present application, the titanium source in step 1) is not strictly limited and can be any titanium source suitable for preparing the titanium silicalite molecular sieve. In a preferred embodiment, in step 1), the titanium source is selected from one or more of an organic titanium source and an inorganic titanium source.

In certain further preferred embodiments, the organic titanium source is selected from at least one of the titanium-containing organic acid esters having the structure shown in the following formula (C):

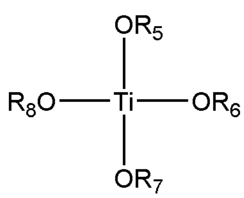
(C);

wherein $R_5$, $R_6$, $R_7$ and $R_8$ are each independently selected from alkyl group having 1 to 6 carbon atoms, preferably selected from linear alkyl group having 1 to 4 carbon atoms or branched alkyl group having 3 to 6 carbon atoms, and further preferably $R_5$, $R_6$, $R_7$ and $R_8$ are each independently selected from linear alkyl group having 2 to 4 carbon atoms or branched alkyl group having 3 to 4 carbon atoms; optionally, $R_5$, $R_6$, $R_7$ and $R_8$ are each independently selected from

methyl, ethyl, n-propyl, isopropyl, n-butyl, sec-butyl, isobutyl, tert-butyl, isopentyl, hexyl or isohexyl; preferably, each independently selected from ethyl, n-propyl, isopropyl, n-butyl, sec-butyl, isobutyl or tert-butyl.

In some further preferred embodiments, the inorganic titanium source is selected from one or more of chlorides, nitrates and sulfates of titanium.

In a particularly preferred embodiment, the titanium source is selected from titanium tetrachloride, titanium sulfate, titanium nitrate, tetraethyl titanate, tetrapropyl titanate and tetrabutyl titanate, or a combination thereof.

In a preferred embodiment, in step 1):

the carbon material used as the hard templating agent is selected from carbon nanoparticles, carbon nanotubes, carbon nanofibers and ordered mesoporous carbon, or a combination thereof;
the resin material used as the hard templating agent is selected from phenolic resin, polyester resin and polyamide resin, or a combination thereof;
the solid inorganic compound used as the hard templating agent is selected from calcium carbonate, magnesium hydroxide and sodium carbonate, or a combination thereof.

The hard templating agent used in the present application can be of the size specifications commonly used in the art. In certain specific embodiments, when the hard templating agent is in the form of particles (such as carbon nanoparticles, ordered mesoporous carbon, calcium carbonate, magnesium hydroxide, etc.), the average particle size of the hard templating agent can be 1-100nm; when the hard templating agent is a carbon nanotube, the diameter of the hard templating agent is 5-50nm and the length is 2-30 μm; when the hard templating agent is a carbon nanofiber, the diameter of the hard templating agent is 3-80nm and the length is 1-40 μm.

In a preferred embodiment, in step 1), the high molecular weight polymer is selected from polyethylene (PE), polypropylene (PP), polyvinyl chloride (PVC), polystyrene (PS), polyvinyl alcohol (PVA), polyvinyl acetate (PVAC), polyacrylonitrile (PAN), polymethyl methacrylate (PMMA), polyvinyl butyral (PVB), polyethyleneimine (PEI), 4-polyvinyl pyridine and poly (diallyl dimethyl ammonium chloride), or a combination thereof.

In a particularly preferred embodiment, the high molecular weight polymer is selected from polyethylene, polypropylene, polystyrene, polyvinyl chloride, polymethyl methacrylate, polyvinyl butyral and polyacrylonitrile, or a combination thereof.

In a preferred embodiment, the weight average molecular weight of the high molecular weight polymer is 10,000-200,000.

In a preferred embodiment, the conditions of the first hydrothermal crystallization treatment in step 2) include: the hydrothermal crystallization temperature is 120-220 °C, the hydrothermal crystallization time is 4-180h; the pressure is autogenous pressure; and the conditions of the first calcination treatment in step 2) include: the calcination temperature is 350-750 °C, and the calcination time is 1-15h.

In a further preferred embodiment, the conditions of the first hydrothermal crystallization treatment in step 2) include: the hydrothermal crystallization temperature is 140-185 °C; the hydrothermal crystallization time is 6-80h; the pressure is autogenous pressure; and the conditions of the first calcination treatment in step 2) include: the calcination temperature is 380-620 °C, and the calcination time is 1.5-4.5h. The titanium silicalite molecular sieve prepared by such preferred embodiment has better catalytic activity.

In certain specific embodiments, in step 2), after the first hydrothermal crystallization treatment, the product of the first hydrothermal crystallization treatment is further subjected to a first filtration treatment, a first drying treatment, and then to the first calcination treatment. Preferably, the temperature of the first drying treatment is 50-120 °C and the time is 1-12h.

In a preferred embodiment, in step 3), the weight ratio of the second templating agent: water: the molecular sieve intermediate is (0.08-3.5): (0.5-25): 1; preferably (0.12-2.5): (2-18): 1. The titanium silicalite molecular sieve prepared by such preferred embodiment has higher catalytic activity.

In a preferred embodiment, the conditions of the second hydrothermal crystallization treatment in step 3) include: the hydrothermal crystallization temperature is 120-220 °C; the hydrothermal crystallization time is 4-180h; the pressure is autogenous pressure; and the conditions of the second calcination treatment in step 3) include: the calcination temperature is 350-750 °C, and the calcination time is 1-15h.

In a further preferred embodiment, the conditions of the second hydrothermal crystallization treatment in step 3) include: the hydrothermal crystallization temperature is 140-185 °C, the hydrothermal crystallization time is 6-80h; the pressure is autogenous pressure; the conditions of the second calcination treatment in step 3) include: the calcination temperature is 380-620 °C, and the calcination time is 1.5-4.5h.

In certain specific embodiments, after the second hydrothermal crystallization treatment, the product of the second hydrothermal crystallization treatment is further subjected to a second filtration treatment, a second drying treatment, and then to the second calcination treatment. Preferably, the temperature of the second drying treatment is 50-120 °C and the time is 1-12h.

In a third aspect, a titanium silicalite molecular sieve having hierarchical porous structure prepared according to

the method described in the second aspect of the present application is provided.

In a fourth aspect, a use of the titanium silicalite molecular sieve having hierarchical porous structure described in the first and third aspects of the present application in catalyzing the gas-phase Beckmann rearrangement reaction of cyclohexanone oxime is provided.

In a fifth aspect, a method for preparing caprolactam from cyclohexanone oxime is provided, comprising the step of subjecting cyclohexanone oxime to a gas-phase Beckmann rearrangement reaction in the presence of the titanium silicalite molecular sieve having hierarchical porous structure described in the first or third aspect of the present application.

In a preferred embodiment, the method comprises the step of contacting and reacting the raw material containing cyclohexanone oxime with the titanium silicalite molecular sieve in a nitrogen atmosphere. Preferably, the raw material comprises cyclohexanone oxime and a solvent, and the solvent is an alcohol, such as methanol, ethanol, butanol, cyclohexanol, etc.

In a further preferred embodiment, the conditions for the gas-phase Beckmann rearrangement reaction include: a reaction temperature of 350-400 °C, a reaction pressure of 0-0.2 MPa, a molar ratio of nitrogen to cyclohexanone oxime of 0.1-30:1, cyclohexanone oxime accounts for 5-50% by weight of the total amount of cyclohexanone oxime and the solvent, and the weight space velocity of cyclohexanone oxime is 1-10 $h^{-1}$.

In certain specific embodiments, the present application provides the following technical solutions:
in one embodiment, the present application provides a titanium silicalite molecular sieve having a hierarchical porous structure, characterized in that the titanium silicalite molecular sieve has the following $^1H$ MAS NMR characteristics:

in the peak separation result of the spectral peaks at the chemical shift in the range of 1-6 ppm of the $^1H$ MAS NMR spectrum of the titanium silicalite molecular sieve:
the peak area of the peak at the chemical shift of 1.8±0.1ppm is recorded as $A_1$, the peak area of the peak at the chemical shift of 2.2ppm±0.1ppm is recorded as $A_2$, the peak area of the peak at the chemical shift of 2.8ppm ±0.1ppm is recorded as $A_3$, the peak area of the peak at the chemical shift of 3.8±0.1ppm is recorded as $A_4$, and the peak area of the peak at the chemical shift of 4.6±0.1ppm is recorded as $A_5$;
$X_1$ as defined by the following formula (1) is any value between 0.5 and 2.2:

$$X_1 = (A_2 + A_3)/A_1, \text{ formula (1)};$$

$X_2$ as defined by the following formula (2) is any value between 0.1 and 1.2:

$$X_2 = A_4 / A_1, \text{ formula (2)};$$

$X_3$ as defined by the following formula (3) is any value between 0.05 and 0.65:

$$X_3 = A_5 / A_1, \text{ formula (3)}.$$

Preferably, the titanium silicalite molecular sieve is characterized in that the value of $X_1$ is any value between 0.6 and 1.8; the value of $X_2$ is any value between 0.15 and 1.10; and the value of $X_3$ is any value between 0.10 and 0.55.

Preferably, the titanium silicalite molecular sieve is characterized in that the molar ratio of silicon atom to titanium atom in the titanium silicalite molecular sieve is (8-150):1, preferably (15-120):1;

Optionally, the configuration of the titanium silicalite molecular sieve is selected from one or more of the MFI topological structure, MEL topological structure, BEA topological structure and SVR topological structure; preferably the MFI topological structure.

Preferably, the titanium silicalite molecular sieve is characterized in that there are multiple cavity structures in the titanium silicalite molecular sieve crystal; wherein the size of a single cavity structure is 5-50 nm, preferably 7-45 nm; further preferably, the volume of all the cavity structures accounts for 8-55% of the total volume of the molecular sieve, and further preferably 12-50%; optionally, the shape of the cavity structure is selected from one or more of a sphere, a cube, an ellipsoid and an irregular cube.

Preferably, the titanium silicalite molecular sieve is characterized in that the titanium silicalite molecular sieve comprises molecular sieve particles composed of a single crystal grain, and/or molecular sieve particles composed of a plurality of crystal grains aggregated; optionally, the molecular sieve particles have an average particle size of 0.1-0.7 μm, preferably 0.13-0.62 μm; a BET specific surface area of 260-620 $m^2/g$, preferably 280-540 $m^2/g$; a micropore specific surface area of 280-500 $m^2/g$, preferably 300-480 $m^2/g$; a total pore volume of 0.16-0.55 $cm^3/g$, preferably 0.22-0.45 $cm^3/g$; a mesopore volume of 0.09-0.38 $cm^3/g$, preferably 0.12-0.34 $cm^3/g$; optionally, a hysteresis loop exists between the

adsorption isotherm and desorption isotherm of low-temperature nitrogen adsorption of the titanium silicalite molecular sieve, preferably, the initial relative pressure (P/P0) at which the hysteresis loop appears is 0.35-0.55.

In another embodiment, the present application provides a method for preparing a titanium silicalite molecular sieve having a hierarchical porous structure, characterized in that it comprises the following steps:

S1) mixing a titanium source, a silicon source, a first templating agent, water, a hard templating agent and a high molecular weight polymer to obtain a reaction mixture;
S2) sequentially performing a first hydrothermal crystallization treatment and a first calcination treatment on the reaction mixture to obtain a molecular sieve intermediate;
S3) mixing the molecular sieve intermediate, a second templating agent and water, and then sequentially performing a second hydrothermal crystallization treatment and a second calcination treatment.

Preferably, the method is characterized in that in step S1), the molar ratio of the titanium source: the silicon source: the first templating agent: water is (0.005-0.55):1:(0.02-1.8):(1-50); preferably (0.01-0.42):1:(0.04-1.5):(10-45); the silicon source is calculated in the form of $SiO_2$, and the weight ratio of the hard templating agent: the high molecular weight polymer: the silicon source is (0.02-0.75):(0.01-0.55):1, preferably (0.04-0.55):(0.03-0.4):1.

Preferably, the method is characterized in that in step S1), the silicon source is selected from at least one of organic silicate, solid silica gel, white carbon black and silica sol; preferably selected from at least one of organic silicate, solid silica gel and white carbon black;

further preferably, the silicon source is organic silicate, and the general formula of the organic silicate is the structure shown in the following formula (A):

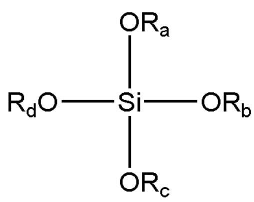

(A);

wherein $R_a$, $R_b$, $R_c$ and $R_d$ are each independently selected from linear or branched alkyl group having 1 to 6 carbon atoms, preferably $R_a$, $R_b$, $R_c$ and $R_d$ are each independently selected from linear alkyl group having 1 to 4 carbon atoms or branched alkyl group having 3 to 4 carbon atoms, and further preferably $R_a$, $R_b$, $R_c$ and $R_d$ are each independently selected from methyl, ethyl, n-propyl, isopropyl, n-butyl, sec-butyl, isobutyl or tert-butyl; further preferably, the organic silicate is selected from one or more of tetramethyl silicate, tetraethyl silicate, tetrabutyl silicate and dimethyl diethyl silicate.

Preferably, the method is characterized in that the first templating agent in step S1) and the second templating agent in step S3) are organic bases; and each is independently preferably at least one selected from quaternary ammonium bases, aliphatic amines and aliphatic alcohol amines;

further preferably, the first templating agent and the second templating agent are each independently selected from at least one quaternary ammonium base having a structure represented by the following formula (B):

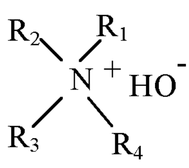

(B);

wherein $R_1$, $R_2$, $R_3$ and $R_4$ are each selected from alkyl group having 1 to 4 carbon atoms, preferably one or more of linear alkyl group having 1 to 4 carbon atoms and branched alkyl group having 3 to 4 carbon atoms, and further preferably $R_1$, $R_2$, $R_3$ and $R_4$ are each selected from one or more of methyl, ethyl, n-propyl, isopropyl, n-butyl, sec-butyl, isobutyl and tert-butyl;
more further preferably, the first templating agent and the second templating agent are each independently tetrapropylammonium hydroxide or a mixture of tetrapropylammonium hydroxide and one or more selected from tetrapropylammonium chloride and tetrapropylammonium bromide.

Preferably, the method is characterized in that in step S1), the titanium source is selected from one or more of an organic titanium source and an inorganic titanium source;

the organic titanium source is a titanium-containing organic acid ester, selected from at least one of the structures represented by the following formula (C):

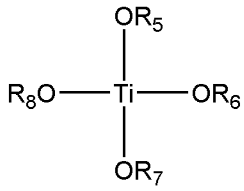

(C);

wherein $R_5$, $R_6$, $R_7$ and $R_8$ are each selected from alkyl group having 1 to 6 carbon atoms, preferably linear alkyl group having 1 to 4 carbon atoms and branched alkyl group having 3 to 6 carbon atoms, and further preferably $R_5$, $R_6$, $R_7$ and $R_8$ are each selected from linear alkyl group having 2 to 4 carbon atoms and branched alkyl group having 2 to 4 carbon atoms; optionally, $R_5$, $R_6$, $R_7$ and $R_8$ are each selected from one of methyl, ethyl, n-propyl, isopropyl, n-butyl, sec-butyl, isobutyl, tert-butyl, isopentyl, hexyl or isohexyl; preferably, each independently selected from one of ethyl, n-propyl, isopropyl, n-butyl, sec-butyl, isobutyl or tert-butyl;
the inorganic titanium source is selected from one or more of chlorides, nitrates or sulfates of titanium;
preferably, the titanium source is selected from one or more of titanium tetrachloride, titanium sulfate, titanium nitrate, tetraethyl titanate, tetrapropyl titanate and tetrabutyl titanate.

Preferably, the method is characterized in that in step S1), the hard templating agent is selected from one or more of a carbon material, a resin material and an inorganic solid compound material; preferably, the carbon material is selected from one or more of carbon nanoparticles, carbon nanotubes, carbon nanofibers and ordered mesoporous carbon; preferably, the resin material is selected from one or more of phenolic resin, polyester resin and polyamide resin; preferably, the inorganic solid compound material is selected from one or more of calcium carbonate, magnesium hydroxide and sodium carbonate.
Preferably, the method is characterized in that in step S1), the high molecular weight polymer is selected from one or more of polyethylene, polypropylene, polyvinyl chloride, polystyrene, polyvinyl alcohol, polyvinyl acetate, polyacrylonitrile, polymethyl methacrylate, polyvinyl butyral, polyethyleneimine, 4-polyvinyl pyridine and poly(diallyldimethylammonium chloride); further preferably, the high molecular weight polymer is selected from one or more of polyethylene, polypropylene, polystyrene and polyacrylonitrile; optionally, the weight average molecular weight of the high molecular weight polymer is 10000-200000.
Preferably, the method is characterized in that step S1) comprises:

a. mixing the titanium source, the silicon source, the first templating agent and water to obtain a hydrolyzed sol of silicon;
b. adding the hard templating agent and the high molecular weight polymer to the hydrolyzed sol of silicon respectively, and mixing them to obtain the reaction mixture;

optionally, the mixing conditions in step a include: stirring at 40-90°C for 6-12 h;
optionally, the mixing conditions in step b include: stirring at 20-50°C for 2-4 h;
preferably, the silicon source is organic silicate, and step a further comprises a hydrolysis and alcohol removal treatment after mixing the silicon source, the first templating agent and water to obtain the hydrolyzed sol of silicon;
optionally, the conditions for the hydrolysis and alcohol removal treatment include: stirring and hydrolyzing at 40-90 °C for 6-12 h; preferably stirring and hydrolyzing at 60-85 °C for 8-10 h.

Preferably, the method is characterized in that in step S3), the weight ratio of the second templating agent: water: the molecular sieve intermediate is ( 0.08-3.5): (0.5-25): 1; preferably (0.12-2.5): (2-18): 1.
Preferably, the method is characterized in that the conditions of the first hydrothermal crystallization treatment in step S2) and the second hydrothermal crystallization treatment in step S3) independently include: the hydrothermal crystallization time is 4-180h, the hydrothermal crystallization temperature is 120-220 °C; preferably, the hydrothermal crystallization time is 6-80h, the hydrothermal crystallization temperature is 140-185 °C; the pressure is autogenous pressure;

the conditions of the first calcination treatment in step S2) and the second calcination treatment in step S3) independently include: a calcination temperature of 350-750 °C and a calcination time of 1-15h; preferably, a calcination temperature of 380-620 °C and a calcination time of 1.5-4.5h.

In another embodiment, a titanium silicalite molecular sieve having a hierarchical porous structure prepared according to the method of the above embodiment is provided.

In another embodiment, a method for preparing caprolactam by gas-phase Beckmann rearrangement of cyclohexanone oxime is provided, comprising: contacting cyclohexanone oxime with a catalyst for reaction, characterized in that the catalyst comprises the titanium silicalite molecular sieve with a hierarchical porous structure according to the above embodiment.

## Examples

The present application is described in detail below through examples, but the present application is not limited to these examples.

In the following examples and comparative examples:

The $^{1}H$ MAS NMR spectrum of the samples were obtained on a Varian Infinity plus-400 spectrometer, using a 4 mm double resonance probe, a $\Phi$4 mm $ZrO_2$ rotor, a resonance frequency of 400.1 MHz, a magic angle speed of 10 kHz, a pulse width of 3.57 $\mu$s, a cycle delay time of 1 s, and a scan number of about 4000 times. The peak separation software was Origin 8.0, and after multi-peak fitting, the peak area and peak intensity were given for calculation processing.

The $^{31}P$ MAS NMR spectrum of the samples were obtained by AVANCE III 600WB NMR spectrometer with trimethylphosphine (TMP) as a probe molecule, using 4 mm double resonance probe, $\Phi$ 4 mm $ZrO_2$ rotor, resonant frequency of 242.9 MHz, magic angle speed of 10 kHz, pulse width of 1.4 $\mu$s, cycle delay time of 1 s, and scan times of about 6000 times.

The X-ray diffraction (XRD) crystal phase diagram of the samples were measured on a Siemens D5005 X-ray diffractometer, the radiation source was K$\alpha$ (Cu), and the test range 2$\theta$ was 0.5° - 70°.

The transmission electron microscopy (TEM) images of the samples were obtained by a Tecnai G2F20S-TWIN transmission electron microscope of FEI company. The cavity structure and size of the sample were obtained according to the TEM electron microscope test, and the volume fraction of the cavity structure volume to the molecular sieve volume was obtained by measuring the TEM images. The specific method is as follows: the volume of each cavity structure in the molecular sieve crystal grain is measured according to the TEM images (the volume of the cavity structure is estimated according to the sphere, wherein the arithmetic mean of the sum of the lengths of the longest line and the shortest line among all lines between two positions on the cavity wall passing through the center of the cavity structure is taken as the spherical diameter, and then the spherical radius is obtained, and then the volume of the corresponding cavity structure is calculated according to the spherical volume formula), the percentage of the sum of the volumes of all the cavity structures in the molecular sieve crystal grains to the volume of the corresponding molecular sieve crystal grains is calculated, and the average value of the volume fractions of the cavity structures of 50 molecular sieve crystal grains is taken as the percentage of the cavity structure volume of the molecular sieve to the volume of the molecular sieve.

The scanning electron microscope (SEM) images of the samples were obtained by a Hitachi S4800 high-resolution cold field emission scanning electron microscope. The average particle size of the samples was obtained by measuring the SEM images, wherein the particle sizes of 50 molecular sieve particles were measured and the arithmetic mean was taken as the average particle size of the molecular sieve.

The total specific surface area and total pore volume of the samples were measured using a ASAP245 static nitrogen adsorption instrument of Micromeritics company according to ASTM D4222-98 standard method.

The BET specific surface area, micropore specific surface area and mesopore volume of the samples were determined by low-temperature nitrogen adsorption-desorption method according to GB/T 19587-2004 standard and calculated using the BET equation.

The adsorption isotherm and desorption isotherm of low temperature nitrogen adsorption of the samples were measured according to ASTM D4222-98 standard method.

The X-ray fluorescence analysis of the samples (determining the molar ratio of silicon to titanium in the molecular sieve) was performed using a 3013 instrument produced by Rigaku Corporation of Japan, with a tungsten target, an excitation voltage of 40 kV, and an excitation current of 250 mA.

In the following examples and comparative examples, unless otherwise specified, all reagents and raw materials used are commercially available products.

**Example 1**

1a) 6 g of tetrabutyl titanate (0.0176 mol), 104 g of tetraethyl silicate (0.5 mol), 65 g of 25 wt% tetrapropylammonium hydroxide (TPAOH, 0.08 mol) aqueous solution and 140 g of water were added to a 500 mL beaker in sequence, placed on a magnetic stirrer with heating and stirring functions to mix evenly, and stirred at 60 °C for 5 hours, and the evaporated water was replenished regularly to obtain a colorless and transparent titanium silica gel solution;

1b) 9 g of carbon nanoparticles (purchased from J&K Chemicals, with an average particle size of 15 nm) and 3 g of polystyrene (PS, purchased from Inotech, with a molecular weight of 20,000) were added to the mixture of step 1a), the weight ratio of the carbon nanoparticles: polystyrene: tetraethyl silicate (calculated as $SiO_2$) is 0.3:0.1:1, and the mixture is stirred for 2 hours;

2) The mixture obtained in step 1b) was transferred to a stainless steel closed reactor, and crystallized isothermally at 170 °C for 24 hours. The obtained product was washed, dried at 110 °C for 3 hours, and then calcined in a muffle furnace at 550 °C for 3 hours to obtain a molecular sieve intermediate M-1. The characterization results of its structural parameters are listed in Table 2. The values of $X_1$-$X_3$ calculated by formula (1)-(3) are listed in Table 3;

3) 10g of the molecular sieve intermediate M-1, 20 g of 25 wt% tetrapropylammonium hydroxide (TPAOH, 5 g) aqueous solution and 40 g of water were uniformly mixed (the weight ratio of the second templating agent (i.e., tetrapropylammonium hydroxide): water: the molecular sieve intermediate was 0.5:5.5:1), transferred to a stainless steel closed reactor, and crystallized isothermally at 170 °C for 24 h. The obtained product was washed, dried at 110 °C for 3 hours, and then calcined at 550 °C in a muffle furnace for 3 hours to obtain a titanium silicalite molecular sieve sample, denoted as C-1, and the characterization results of its structural parameters are listed in Table 2.

The $^1$H MAS NMR spectrum of the molecular sieve C-1 is shown in FIG1. It can be seen from the figure that after peak separation, the titanium silicalite molecular sieve shows a peak of isolated silanol group at a chemical shift of 1.8 ppm, and peaks of silanol group with hydrogen bond interaction in a chemical shift range of 2-6 ppm, at 2.2 ppm, 2.8 ppm, 3.8 ppm and 4.6 ppm, respectively, indicating that the titanium silicalite molecular sieve has abundant of silanol group active species. The peak areas of the above five peaks obtained by integral calculation are: $A_1$ is 77559, $A_2$ is 45005, $A_3$ is 39245, $A_4$ is 30954, and As is 19694. The values of $X_1$-$X_3$ calculated by formula (1)-(3) are listed in Table 3.

The $^{31}$P-TMP MAS NMR spectrum of the molecular sieve C-1 is shown in FIG 2, wherein there is a spectral peak corresponding to Brönsted acid at a chemical shift of -5 ± 1 ppm, and there is a spectral peak corresponding to Lewis acid at a chemical shift of -34 ± 1 ppm, indicating that the molecular sieve has both Brönsted acid and Lewis acid properties; wherein the peak intensity $N_1$ is 23597, $N_2$ is 54809, $N_3$ is 326304, and the values of $Y_1$ and $Y_2$ calculated by formula (4)-(5) are listed in Table 3.

The XRD spectrum of the molecular sieve C-1 is shown in FIG 3, indicating that the titanium silicalite molecular sieve has an MFI topological structure.

The TEM electron microscope image of the molecular sieve C-1 is shown in FIG 4. It can be seen from the figure that the crystal grains of the molecular sieve have hierarchical porous structures with multiple cavity structures inside, and after measurement and calculation, the size of each cavity structure is in the range of 5-41nm.

The SEM electron microscope image of the molecular sieve C-1 is shown in FIG 5. It can be seen from the figure that the molecular sieve is uniform ellipsoidal particles.

The low temperature nitrogen adsorption test result of the molecular sieve C-1 is shown in FIG 6. It can be seen that there is an obvious hysteresis loop between the nitrogen adsorption isotherm and the desorption isotherm. The initial relative pressure ($P/P_0$) at which the hysteresis loop appears is 0.46.

The TEM image of the molecular sieve intermediate M-1 that has not undergone the dissolution and recrystallization process (i.e., the second hydrothermal crystallization treatment) of step 3) is shown in FIG 7. Comparing FIG 7 with FIG 4, it can be seen that the molecular sieve intermediate M-1 has formed a large number of cavity structures within the grains after the second hydrothermal crystallization treatment of step 3).

The $^1$H MAS NMR spectrum of the molecular sieve intermediate M-1 is shown in FIG 8. It can be seen from the figure that after peak separation, the titanium silicalite molecular sieve shows a peak of isolated silanol group at a chemical shift of 1.8 ppm, and peaks of silanol group with hydrogen bond interaction in a chemical shift range of 2-6 ppm, at 2.2 ppm, 2.8 ppm, 3.8 ppm and 4.6 ppm, respectively. The peak areas of the above five peaks obtained by integral calculation are: $A_1$ is 31578, $A_2$ is 0, $A_3$ is 61089, $A_4$ is 142592, and As is 61428. The calculated values of $X_1$-$X_3$ are listed in Table 3.

The $^{31}$P-TMP MAS NMR spectrum of the molecular sieve intermediate M-1 is shown in FIG 9, wherein there is a spectral peak corresponding to Brönsted acid at a chemical shift of -5 ± 1 ppm, and there is a spectral peak corresponding to Lewis acid at a chemical shift of -34 ± 1 ppm; wherein the peak intensity $N_1$ is 163607, $N_2$ is 24923, and $N_3$ is 367232, and the calculated values of $Y_1$ and $Y_2$ are listed in Table 3.

**Comparative Example 1**

The titanium silicalite molecular sieve was prepared according to the method of Example 1, except that no hard templating agent carbon nanoparticles and high molecular weight polymer polystyrene were added. The specific preparation conditions are listed in Table 1. The obtained molecular sieve was denoted as D-1, and the characterization results of its structural parameters are listed in Table 2, and the calculated values of $X_1$-$X_3$ and $Y_1$-$Y_2$ are listed in Table 3.

**Comparative Example 2**

This comparative example prepares the titanium silicalite molecular sieve according to the method disclosed in the prior art CN112744836A, and the specific steps are as follows:

1) 25 wt% aqueous solution of tetrapropylammonium hydroxide (TPAOH), tetraethyl orthosilicate (TEOS), tetrabutyl titanate (TBOT) and deionized water were weighted in a molar ratio of TPAOH: TEOS: TBOT: $H_2O$ of 0.2: 1: 0.015: 100 and added into a beaker in sequence, placed on a magnetic stirrer with heating and stirring functions to mix evenly, and stirred at 80 °C for 3 hours for a first hydrolyzing, and the evaporated water was replenished regularly to obtain a colorless transparent hydrolyzate, i.e., the first hydrolysis mixture.
2) Activated carbon was added to the first hydrolysis mixture during stirring, wherein the mass ratio of $SiO_2$ to semi coke-based activated carbon is 1:0.16. The mixture was transferred to a stainless steel closed reactor and subjected to a first hydrothermal treatment at 170°C for 24 hours. After filtering and washing the product, the filter cake was dried at 110°C for 24 hours and then calcined at 550°C for 6 hours to obtain an intermediate titanium silicalite molecular sieve denoted as HS-1.
3) 25 wt% aqueous solution of tetrapropylammonium hydroxide (TPAOH), tetraethyl orthosilicate (TEOS), tetrabutyl titanate (TBOT) and deionized water were weighted in a molar ratio of TPAOH: TEOS: TBOT: $H_2O$ of 2: 20: 1: 55 and added into a beaker in sequence, placed on a magnetic stirrer with heating and stirring functions to mix evenly, and stirred at 70 °C for 10 hours for a second hydrolyzing, and the evaporated water was replenished regularly to obtain a colorless transparent hydrolyzate, i.e., the second hydrolysis mixture.
(4) The above intermediate titanium silicalite molecular sieve HS-1, the second hydrolysis mixture and ammonium chloride were mixed to obtain a mixed material, in which the molar ratio of $TiO_2$, $SiO_2$ and $NH_4^+$ was 1:35:0.3. The mixed material was transferred to a stainless steel reactor and subjected to a second hydrothermal treatment at 170°C for 24 hours, filtered, washed, dried at 120°C for 24 hours, and calcined at 550°C for 6 hours to obtain a molecular sieve product denoted as D-2.

The TEM image of the molecular sieve D-2 is shown in FIG 10. Comparing FIG 10 with FIG 4, it can be seen that the molecular sieve C-1 prepared in Example 1 has an obvious multi-cavity structure within the crystal grains compared with the molecular sieve D-2.
The $^{1}H$ MAS NMR spectrum of the molecular sieve D-2 is shown in FIG11, from which it can be seen that $A_1$ is 123510, $A_2$ is 60045, $A_3$ is 0, $A_4$ is 71389, $A_5$ is 40722, and the calculated values of $X_1$-$X_3$ are listed in Table 3.
The $^{31}P$-TMP MAS NMR spectrum of the molecular sieve D-2 is shown in FIG12, wherein the peak intensity $N_1$ is 4387, $N_2$ is 15086, and $N_3$ is 1710413. The calculated values of $Y_1$ and $Y_2$ are listed in Table 3.

**Comparative Example 3**

The titanium silicalite molecular sieve was prepared according to the method of Example 1, except that no hard templating agent carbon nanoparticles were added. The specific preparation conditions are listed in Table 1. The obtained molecular sieve was denoted as D-3, and the characterization results of its structural parameters are listed in Table 2, and the calculated values of $X_1$-$X_3$ and $Y_1$-$Y_2$ are listed in Table 3.

**Examples 2-9**

The titanium silicalite molecular sieves were prepared according to the method of Example 1, except that the ratios and synthesis conditions were changed as shown in Table 1. The obtained titanium silicalite molecular sieves were respectively denoted as C-2 to C-9, and the characterization results of their structural parameters are listed in Table 2, and the calculated values of $X_1$-$X_3$ and $Y_1$-$Y_2$ are listed in Table 3.

**Example 10**

The titanium silicalite molecular sieve was prepared according to the method of Example 1, except that the

templating agent was changed as shown in Table 1, wherein the templating agent tetrapropylammonium hydroxide used in step 1a) and step 3) was replaced by tetrabutylammonium hydroxide (TBAOH), and the specific preparation conditions are listed in Table 1. The obtained titanium silicalite molecular sieve was denoted as C-10, and the characterization results of its structural parameters are listed in Table 2, and the calculated values of $X_1$-$X_3$ and $Y_1$-$Y_2$ are listed in Table 3.

The XRD spectrum of the molecular sieve C-10 is shown in FIG 13, indicating that the molecular sieve has a MEL topological structure.

**Example 11**

The titanium silicalite molecular sieve was prepared according to the method of Example 1, except that the ratios and the templating agent were changed as shown in Table 1, wherein the templating agent tetrapropylammonium hydroxide used in step 1a) and step 3) was replaced by tetrabutylammonium hydroxide (TBAOH), and the specific preparation conditions are listed in Table 1. The obtained titanium silicalite molecular sieve was denoted as C-11, and the characterization results of its structural parameters are listed in Table 2, and the calculated values of $X_1$-$X_3$ and $Y_1$-$Y_2$ are listed in Table 3.

The XRD spectrum of the molecular sieve C-11 is shown in FIG 14, indicating that the molecular sieve has a BEA topological structure.

**Example 12**

The titanium silicalite molecular sieve was prepared according to the method of Example 1, except that:

the temperature of the first hydrothermal crystallization treatment was 120°C and the time was 96h; the temperature of the first calcination treatment was 350°C and the time was 8h;
the temperature of the second hydrothermal crystallization treatment was 120°C and the time was 96h; the temperature of the second calcination treatment was 350°C and the time was 8h;
the obtained titanium silicalite molecular sieve sample was denoted as C-12; the characterization results of the obtained molecular sieve C-12 are listed in Table 2.

**Examples 13-15**

The titanium silicalite molecular sieves were prepared according to the method of Example 1, except that the ratios, the silicon source and the templating agent were changed as shown in Table 1. The obtained titanium silicalite molecular sieves were respectively denoted as C-13 to C-15, and the characterization results of their structural parameters are listed in Table 2, and the calculated values of $X_1$-$X_3$ and $Y_1$-$Y_2$ are listed in Table 3.

Table 1 Operating conditions of the examples and comparative examples

| Molecular sieve number | First templating agent / second templating agent ( showing only one if both are the same) | First templating agent/silicon source (molar ratio) | Silicon source | Titanium source | Titanium source/silicon source (molar ratio) | Water/silicon source (molar ratio) | Hard templating agent | High molecular weight polymer | Hard templating agent : high molecular weight polymer : silicon source (mass ratio) | Second templating agent : water : molecular sieve intermediate (mass ratio) |
|---|---|---|---|---|---|---|---|---|---|---|
| C-1 | TPAOH | 0.16 | tetraethyl silicate | tetrabutyl titanate | 0.035 | 21 | carbon nanoparticles | PS | 0.3:0.1:1 | 0.5:5.5:1 |
| C-2 | TPAOH | 0.08 | tetraethyl silicate | tetrabutyl titanate | 0.012 | 15 | carbon nanotubes | PS | 0.4:0.15:1 | 0.56:7.14:1 |
| C-3 | TPAOH | 0.20 | tetraethyl silicate | tetraethyl titanate | 0.015 | 12 | phenolic resin | PVC | 0.5:0.25:1 | 0.67:5.5:1 |
| C-4 | TPAOH | 0.12 | tetraethyl silicate | tetrapropyl titanate | 0.025 | 21 | carbon nanofibers | PS | 0.4:0.35:1 | 0.45:4.17:1 |
| C-5 | TPAOH | 0.16 | tetraethyl silicate | titanium tetrachloride | 0.020 | 15 | calcium carbonate | PMMA | 0.7:0.1:1 | 0.83:5.5:1 |
| C-6 | TPAOH | 0.50 | tetraethyl silicate | tetrabutyl titanate | 0.055 | 8 | carbon nanoparticles | PVB | 0.3:0.2:1 | 1.25:6.67:1 |
| C-7 | TPAOH | 0.12 | tetraethyl silicate | tetrabutyl titanate | 0.045 | 12 | ordered mesoporous carbon | PS | 0.3:0.3:1 | 2.00:5.00:1 |
| C-8 | TPAOH | 0.16 | tetraethyl silicate | tetrabutyl titanate | 0.040 | 25 | carbon nanoparticles | PVB | 0.3:0.4:1 | 2.00:5.5:1 |
| C-9 | TPAOH | 1.80 | tetraethyl silicate | tetrabutyl titanate | 0.550 | 50 | carbon nanoparticles | PS | 0.75:0.55:1 | 0.1:20:1 |
| C-10 | TBAOH | 0.16 | tetraethyl silicate | tetrabutyl titanate | 0.035 | 21 | carbon nanoparticles | PS | 0.3:0.1:1 | 0.5:5.5:1 |
| C-11 | TEAOH | 0.16 | tetraethyl silicate | tetrabutyl titanate | 0.015 | 21 | carbon nanoparticles | PS | 0.3:0.1:1 | 0.5:5.5:1 |

(continued)

| Molecular sieve number | First templating agent / second templating agent ( showing only one if both are the same) | First templating agent/silicon source (molar ratio) | Silicon source | Titanium source | Titanium source/silicon source (molar ratio) | Water/silicon source (molar ratio) | Hard templating agent | High molecular weight polymer | Hard templating agent : high molecular weight polymer : silicon source (mass ratio) | Second templating agent : water : molecular sieve intermediate (mass ratio) |
|---|---|---|---|---|---|---|---|---|---|---|
| C-12 | TPAOH | 0.16 | tetraethyl silicate | tetrabutyl titanate | 0.035 | 21 | carbon nanoparticles | PS | 0.3:0.1:1 | 0.5:5.5:1 |
| C-13 | Tetrapropylammonium chloride | 0.16 | dimethyl diethyl silicate | tetrabutyl titanate | 0.035 | 21 | carbon nanoparticles | PS | 0.35:0.45:1 | 0.7:5.2:1 |
| C-14 | Tetrapropyl ammonium bromide | 0.16 | white carbon black | tetrabutyl titanate | 0.035 | 21 | carbon nanoparticles | PS | 0.55:0.35:1 | 1.2:4.5:1 |
| C-15 | TPAOH/TBAOH | 0.16 | white carbon black | tetrabutyl titanate | 0.035 | 21 | carbon nanoparticles | PS | 0.45:0.55:1 | 1.8:2.5:1 |
| D-1 | TPAOH | 0.16 | tetraethyl silicate | tetrabutyl titanate | 0.035 | 21 | - | - | - | 0.50:5.5:1 |
| D-2 | TPAOH | 0.20 | tetraethyl silicate | tetrabutyl titanate | 0.015 | 100 | semi coke-based activated carbon | - | 0.16:0:1 | - |
| D-3 | TPAOH | 0.16 | tetraethyl silicate | tetrabutyl titanate | 0.035 | 21 | - | PS | 0:0.1:1 | 0.5:5.5:1 |

In Table 1, TPAOH is tetrapropylammonium hydroxide, TBAOH is tetrabutylammonium hydroxide, TEAOH is tetraethylammonium hydroxide, PVC is polyvinyl chloride (weight average molecular weight 15000), PS is polystyrene (weight average molecular weight 20000), PMMA is polymethyl methacrylate (weight average molecular weight 18000), PVB is polyvinyl butyral (weight average molecular weight 22000); the average particle size of carbon nanoparticles is 50 nm, the diameter of carbon nanotubes is 20 nm, and the length is 15 μm; the diameter of carbon nanofibers is 60 nm, and the length is 30 μm. The average particle size of ordered mesoporous carbon is 10 nm, the average particle size of phenolic resin is 30 nm, and the average particle size of calcium carbonate is 20 nm.

In Table 1, the water in the calculation of "water/silicon source" in the examples of the present application also includes water from the first templating agent aqueous solution; the water in the calculation of "water/molecular sieve intermediate" also includes water from the second templating agent aqueous solution

Table 2 Properties of the molecular sieves obtained in the examples and comparative examples

| Molecular sieve number | Molar ratio of silicon atom to titanium atom | Average particle size of molecular sieve particles/μm | BET specific surface area/ $m^2/g$ | Micropore specific surface area/ $m^2/g$ | Total pore volume / $cm^3/g$ | Micropore volume / $cm^3/g$ | Mesopore volume / $cm^3/g$ | Molecular sieve structure | Radial length of cavity structure*/nm | Percentage of cavity structur e to the total volume of molecular sieve/% | P/Po |
|---|---|---|---|---|---|---|---|---|---|---|---|
| C-1 | 33 | 0.42 | 464 | 380 | 0.419 | 0.109 | 0.310 | MFI | 5-38 | 45 | 0.46 |
| C-2 | 80 | 0.47 | 504 | 370 | 0.425 | 0.119 | 0.306 | MFI | 7-35 | 36 | 0.42 |
| C-3 | 69 | 0.43 | 538 | 383 | 0.427 | 0.093 | 0.334 | MFI | 7-32 | 41 | 0.41 |
| C-4 | 44 | 0.37 | 400 | 318 | 0.412 | 0.093 | 0.319 | MFI | 5-39 | 32 | 0.39 |
| C-5 | 53 | 0.44 | 416 | 333 | 0.407 | 0.067 | 0.340 | MFI | 8-24 | 28 | 0.40 |
| C-6 | 22 | 0.45 | 505 | 356 | 0.387 | 0.066 | 0.321 | MFI | 5-26 | 41 | 0.45 |
| C-7 | 27 | 0.48 | 536 | 355 | 0.399 | 0.075 | 0.324 | MFI | 7-34 | 46 | 0.44 |
| C-8 | 29 | 0.44 | 418 | 367 | 0.341 | 0.025 | 0.316 | MFI | 8-26 | 38 | 0.44 |
| C-9 | 12 | 0.39 | 435 | 323 | 0.385 | 0.089 | 0.296 | MFI | 12-35 | 11 | 0.43 |
| C-10 | 33 | 0.42 | 424 | 310 | 0.407 | 0.133 | 0.274 | MEL | 13-41 | 36 | 0.38 |
| C-11 | 71 | 0.47 | 493 | 307 | 0.424 | 0.110 | 0.314 | BEA | 5-38 | 25 | 0.41 |
| C-12 | 32 | 0.34 | 475 | 352 | 0.405 | 0.093 | 0.312 | MFI | 4-32 | 16 | 0.39 |
| C-13 | 32 | 0.43 | 463 | 342 | 0.487 | 0.332 | 0.155 | MFI | 12-25 | 23 | 0.38 |
| C-14 | 33 | 0.47 | 475 | 338 | 0.473 | 0.313 | 0.16 | MFI | 15-31 | 25 | 0.41 |
| C-15 | 31 | 0.45 | 482 | 352 | 0.451 | 0.321 | 0.13 | MFI | 15-35 | 31 | 0.43 |
| M-1 | 35 | 0.46 | 436 | 312 | 0.446 | 0.314 | 0.132 | MFI | 14-22 | 19 | 0.40 |
| D-1 | 30 | 0.44 | 426 | 331 | 0.381 | 0.177 | 0.204 | MFI | 1-5 | 7 | 0.42 |
| D-2 | 71 | 0.43 | 381 | 338 | 0.365 | 0.150 | 0.215 | MFI | 1-4 | 9 | 0.43 |
| D-3 | 34 | 0.38 | 374 | 304 | 0.358 | 0.192 | 0.166 | MFI | 3-5 | 8 | 0.42 |

*The radial length of the cavity structure shown in the table is the length of the line between any two positions on the cavity wall of the measured cavity structure passing through the center of the cavity structure.

According to the data in Table 2 above, it can be seen that compared with the molecular sieves D-1 to D-3 prepared in Comparative Examples 1-3, the molecular sieves C-1 to C-15 prepared by the method of the present application have a larger cavity size and a larger percentage of the total cavity structure to the total volume of molecular sieve.

**Test Case**

The molecular sieves prepared in the above examples and comparative examples were evaluated. The prepared molecular sieves were tableted and then crushed. The particles with a size of 20-60 mesh were used as catalysts for the gas-phase Beckmann rearrangement reaction of cyclohexanone oxime. The catalytic performance of the obtained molecular sieves was evaluated. The evaluation conditions were as follows:
the evaluation device is a normal pressure continuous flow fixed bed reactor with an inner diameter of 5 mm and a catalyst loading of 2 g. After loading the catalyst, it was pretreated in a nitrogen atmosphere at normal pressure and 350°C for 3 hours. The concentration of the raw material cyclohexanone oxime was 35% by weight, and the solvent was methanol. The reaction conditions included: the cyclohexanone oxime weight space velocity (WHSV, cyclohexanone oxime flow rate in the feed/catalyst weight in the reactor) was 2 $h^{-1}$, the reaction temperature was 380°C, the nitrogen flow rate was 4 L/h, and the reaction time was 24 h and 200 h, respectively.

The reaction products were collected after cooling, and the concentrations of various substances were quantitatively analyzed using gas chromatography, using a 6890 gas chromatograph produced by Agilent, and an HP-5 column for analytical chromatography. The test conditions included: a vaporization chamber temperature of 250°C, a detection chamber temperature of 230°C, a column temperature of programmed temperature, a constant temperature of 110°C for 8 minutes, a temperature increase of 15°C/min to 230°C, and a constant temperature for 14 minutes; the results are listed in Table 3 below.
wherein:

cyclohexanone oxime conversion rate (mol%) = (molar content of cyclohexanone oxime in the feed - molar content of cyclohexanone oxime in the product)/molar content of cyclohexanone oxime in the feed × 100%;

caprolactam selectivity (mol%) = molar percentage of caprolactam in the product/(100-molar percentage of cyclohexanone oxime in the product) × 100%;

cyclohexanone oxime conversion rate reduction rate (%) = (cyclohexanone conversion rate for 24h- cyclohexanone conversion rate for 200h)/cyclohexanone conversion rate for 24h × 100%;

caprolactam selectivity reduction rate (%) = (caprolactam selectivity for 24h - caprolactam selectivity for 200h)/aprolactam selectivity for 24h × 100%.

Table 3 Test results of the molecular sieves obtained in examples and comparative examples

| Molecular sieve number | $X_1$ | $X_2$ | $X_3$ | $Y_1$ | $Y_2$ | Reaction for 24h | | Reaction for 200 h | | Cyclohexanone oxime conversion rate reduction rate (%) | Caprolactam selectivity reduction rate (%) |
|---|---|---|---|---|---|---|---|---|---|---|---|
| | | | | | | Cyclohexanone oxime conversion rate/mol% | Caprolactam selectivity/mol % | Cyclohexanone oxime conversion rate/mol% | Caprolactam selectivity/mol % | | |
| C-1 | 1.08 | 0.40 | 0.25 | 0.07 | 0.17 | 99.5 | 96.9 | 91.5 | 92.4 | 8.0 | 4.6 |
| C-2 | 0.85 | 1.02 | 0.37 | 0.05 | 0.13 | 99.2 | 97.7 | 92.5 | 91.3 | 6.8 | 6.6 |
| C-3 | 0.63 | 1.09 | 0.32 | 0.06 | 0.17 | 99.7 | 94.0 | 91.7 | 90.5 | 8.0 | 3.7 |
| C-4 | 1.49 | 0.32 | 0.18 | 0.08 | 0.19 | 99.5 | 96.8 | 92.5 | 91.6 | 7.0 | 5.4 |
| C-5 | 0.84 | 0.96 | 0.26 | 0.07 | 0.22 | 99.1 | 95.0 | 92.6 | 91.5 | 6.6 | 3.7 |
| C-6 | 0.67 | 0.31 | 0.36 | 0.09 | 0.24 | 99.0 | 95.6 | 93.4 | 90.6 | 5.7 | 5.2 |
| C-7 | 1.46 | 0.27 | 0.12 | 0.11 | 0.25 | 98.7 | 90.5 | 92.6 | 92.1 | 6.2 | 4.6 |
| C-8 | 0.80 | 0.56 | 0.23 | 0.12 | 0.24 | 99.5 | 95.1 | 94.1 | 92.3 | 5.4 | 2.9 |
| C-9 | 0.52 | 0.11 | 0.06 | 0.02 | 0.21 | 95.4 | 92.1 | 82.5 | 83.7 | 13.5 | 9.1 |
| C-10 | 0.77 | 0.95 | 0.16 | 0.03 | 0.23 | 99.4 | 93.9 | 92.4 | 89.6 | 7.0 | 4.6 |
| C-11 | 1.35 | 0.65 | 0.42 | 0.02 | 0.24 | 99.1 | 94.6 | 93.6 | 89.8 | 5.5 | 5.1 |
| C-12 | 0.51 | 0.12 | 0.08 | 0.04 | 0.17 | 92.3 | 91.1 | 81.5 | 80.5 | 11.7 | 11.6 |
| C-13 | 0.85 | 0.23 | 0.13 | 0.05 | 0.12 | 99.3 | 95.6 | 92.2 | 87.5 | 7.2 | 8.5 |
| C-14 | 0.73 | 0.41 | 0.22 | 0.04 | 0.14 | 98.7 | 97.2 | 90.7 | 86.1 | 8.1 | 11.4 |
| C-15 | 1.05 | 0.37 | 0.35 | 0.08 | 0.16 | 99.1 | 93.2 | 92.3 | 83.8 | 6.9 | 10.1 |
| M-1 | 1.93 | 4.52 | 1.94 | 0.45 | 0.07 | 90.5 | 94.3 | 77.6 | 72.4 | 14.3 | 23.2 |
| D-1 | 0.48 | 0.58 | 0.32 | 0.002 | 0.009 | 87.8 | 88.0 | 65.6 | 72.1 | 25.3 | 18.1 |
| D-2 | 0.27 | 0.75 | 0.03 | 0.004 | 0.05 | 89.1 | 87.5 | 69.7 | 70.6 | 21.8 | 19.3 |
| D-3 | 0.34 | 0.54 | 0.04 | 0.003 | 0.07 | 88.6 | 86.5 | 62.3 | 68.4 | 29.7 | 20.9 |

According to the data in Table 3 above, it can be seen that:
compared with the molecular sieves D-1 to D-3 prepared in Comparative Examples 1-3, the molecular sieves C-1 to C-15 prepared by the method of the present application have $^1H$ MAS NMR spectrums in which $X_1$ is in the range of 0.5-2.2, $X_2$ is in the range of 0.1-1.2, and $X_3$ is in the range of 0.05-0.65. The molecular sieves C-1 to C-15 have higher catalytic activity in the gas-phase Beckmann rearrangement reaction of cyclohexanone oxime, higher cyclohexanone oxime conversion rate and caprolactam selectivity, and the cyclohexanone oxime conversion rate reduction rate and caprolactam selectivity reduction rate under long-term reaction conditions (200h) are lower, indicating that the molecular sieves C-1 to C-15 provided in the present application have higher catalytic stability.

Furthermore, the molecular sieves C-1 to C-8, C-10 to C-11 and C-13 to C-15 prepared in the examples have $X_1$ in the range of 0.6-1.8, $X_2$ in the range of 0.15-1.10, and $X_3$ in the range of 0.10-0.55. Compared with the molecular sieves C-9 and C-12, the cyclohexanone oxime conversion rate and caprolactam selectivity in the reaction using molecular sieves C-1 to C-8, C-10 to C-11 and C-13 to C-15 are higher, and the catalytic stability under long-term reaction conditions (200h) is higher.

Comparing Example 1 with Example 9, Example 1 prepared the molecular sieve according to the raw material addition ratio in the preferred embodiment, and the obtained molecular sieve C-1 had higher cyclohexanone oxime conversion rate and caprolactam selectivity in the catalytic reaction, and higher catalytic stability under long-term reaction conditions (200h).

Comparing Example 1 with Example 12, Example 1 prepared the molecular sieve according to the reaction conditions in the preferred embodiment, and the obtained molecular sieve C-1 had higher cyclohexanone oxime conversion rate and caprolactam selectivity in the catalytic reaction, and higher catalytic stability under long-term reaction conditions (200h).

The preferred embodiments of the present application are described in detail above; however, the present application is not limited to the specific details in the above embodiments. Within the technical concept of the present application, a variety of simple modifications can be made to the technical solution of the present application, and these simple modifications all fall within the protection scope of the present application.

It should also be noted that the various specific technical features described in the above specific embodiments can be combined in any suitable manner without contradiction. In order to avoid unnecessary repetition, the present application will not further describe various possible combinations.

In addition, the various embodiments of the present application may be arbitrarily combined, and as long as they do not violate the concept of the present application, they should also be regarded as the contents disclosed in the present application.

## Claims

1. A titanium silicalite molecular sieve with a hierarchical porous structure, wherein the spectral peaks of the $^1H$ MAS NMR spectrum of the titanium silicalite molecular sieve in the chemical shift range of 1-6 ppm are separated to obtain five spectral peaks whose chemical shifts corresponding to the peak positions are 1.8±0.1 ppm, 2.2 ppm±0.1 ppm, 2.8 ppm±0.1 ppm, 3.8±0.1 ppm and 4.6±0.1 ppm, respectively, wherein:
the peak area of the spectral peak whose peak is at 1.8±0.1 ppm is recorded as $A_1$, the peak area of the spectral peak whose peak is at 2.2 ppm±0.1 ppm is recorded as $A_2$, the peak area of the spectral peak whose peak is at 2.8 ppm±0.1 ppm is recorded as $A_3$, the peak area of the spectral peak whose peak is at 3.8±0.1 ppm is recorded as $A_4$, and the peak area of the spectral peak whose peak is at 4.6±0.1 ppm is recorded as $A_5$, then:

$X_1$ as defined by the following formula (1) is in the range of 0.5-2.2, preferably in the range of 0.6-1.8:

$$X_1 = (A_2 + A_3)/A_1 \qquad (1);$$

$X_2$ as defined by the following formula (2) is in the range of 0.1-1.2, preferably in the range of 0.15-1.10:

$$X_2 = A_4/A_1 \qquad (2);$$

$X_3$ as defined by the following formula (3) is in the range of 0.05-0.65, preferably in the range of 0.10-0.55:

$$X_3 = A_5/A_1 \qquad (3).$$

2. The titanium silicalite molecular sieve according to claim 1, wherein the solid nuclear magnetic resonance characterization is performed using trimethylphosphine (TMP) as a probe molecule, and the $^{31}P$ MAS NMR spectrum of the molecular sieve shows three characteristic peaks at chemical shifts of -5±1 ppm, -34±1 ppm and -61±1 ppm, respectively, wherein the peak intensity of the characteristic peak at the chemical shift of -5±1 ppm is recorded as $N_1$, the peak intensity of the characteristic peak at the chemical shift of -34±1 ppm is recorded as $N_2$, and the peak intensity of the characteristic peak at the chemical shift of -61±1 ppm is recorded as $N_3$, then:

$Y_1$ as defined by the following formula (4) is in the range of 0.005-0.15, preferably in the range of 0.02-0.12:

$$Y_1 = N_1 / N_3 \qquad (4);$$

and
$Y_2$ as defined by the following formula (5) is in the range of 0.10-0.30, preferably in the range of 0. 1-0.26:

$$Y_2 = N_2 / N_3 \qquad (5).$$

3. The titanium silicalite molecular sieve according to claim 1 or 2, wherein the molar ratio of silicon atom to titanium atom in the titanium silicalite molecular sieve is (8-150): 1, preferably (15-120): 1;
optionally, the configuration of the titanium silicalite molecular sieve is selected from the group consisting of MFI topological structure, MEL topological structure, BEA topological structure and SVR topological structure; preferably, MFI topological structure.

4. The titanium silicalite molecular sieve according to any one of claims 1 to 3, wherein the titanium silicalite molecular sieve has a plurality of cavity structures in the crystal grains; and the size of the cavity structure is in the range of 5-50 nm, preferably in the range of 7-45 nm;

preferably, the cavity structure volume of the molecular sieve accounts for 8-55% of the volume of the molecular sieve, and further preferably 12-50%;
optionally, the shape of the cavity structure is selected from a sphere, an ellipsoid, a cylinder, a polyhedron and an irregular shape, or a combination thereof.

5. The titanium silicalite molecular sieve according to any one of claims 1 to 4, wherein the titanium silicalite molecular sieve includes molecular sieve particles composed of a single crystal grain, and/or molecular sieve particles composed of a plurality of crystal grains aggregated;
optionally, the average particle size of the molecular sieve particles is 0.1-0.7 μm, preferably 0.13-0.62 μm; the BET specific surface area is 260-620 $m^2/g$, preferably 280-540 $m^2/g$; the micropore specific surface area is 280-500 $m^2/g$, preferably 300-480 $m^2/g$; the total pore volume is 0.16-0.55 $cm^3/g$, preferably 0.22-0.45 $cm^3/g$; the mesopore volume is 0.09-0.38 $cm^3/g$, preferably 0.12-0.34 $cm^3/g$.

6. The titanium silicalite molecular sieve according to any one of claims 1 to 5, wherein a hysteresis loop exists between the adsorption isotherm and the desorption isotherm of low temperature nitrogen adsorption of the titanium silicalite molecular sieve;
preferably, the initial relative pressure ($P/P_0$) at which the hysteresis loop appears is in the range of 0.35-0.55, preferably in the range of 0.38-0.48.

7. A method for preparing the titanium silicalite molecular sieve with a hierarchical porous structure according to any one of claims 1 to 6, comprising the following steps:

1) mixing a titanium source, a silicon source, a first templating agent, water, a hard templating agent and a high molecular weight polymer to obtain a reaction mixture, wherein the hard templating agent is selected from one or more of a carbon material, a resin material and a solid inorganic compound;
2) sequentially performing a first hydrothermal crystallization treatment and a first calcination treatment on the reaction mixture to obtain a molecular sieve intermediate;
3) mixing the molecular sieve intermediate, a second templating agent and water, and then sequentially performing a second hydrothermal crystallization treatment and a second calcination treatment,

preferably, the first templating agent and the second templating agent are organic bases, and are independently selected from quaternary ammonium bases, aliphatic amines, aliphatic alcohol amines, or a combination thereof.

**8.** The method according to claim 7, wherein in step 1), the molar ratio of the titanium source: the silicon source: the first templating agent: water is (0.005-0.55): 1: (0.02-1.8): (1-50); preferably (0.01-0.42): 1: (0.04-1.5): (10-45); the weight ratio of the hard templating agent: the high molecular weight polymer: the silicon source (calculated as $SiO_2$) is (0.02-0.75): (0.01-0.55): 1, preferably (0.04-0.55): (0.03-0.4): 1.

**9.** The method according to claim 7 or 8, wherein in step 1), the silicon source is selected from organic silicate, solid silica gel, white carbon black and silica sol, or a combination thereof; preferably selected from organic silicate, solid silica gel and white carbon black, or a combination thereof;

further preferably selected from at least one of the organic silicates having the structure shown in the following formula (A):

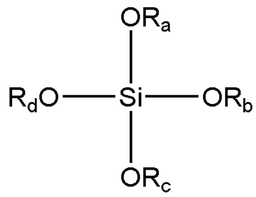
(A);

wherein $R_a$, $R_b$, $R_c$ and $R_d$ are each independently selected from linear or branched alkyl group having 1 to 6 carbon atoms, preferably $R_a$, $R_b$, $R_c$ and $R_d$ are each independently selected from linear alkyl group having 1 to 4 carbon atoms or branched alkyl group having 3 to 4 carbon atoms, and further preferably $R_a$, $R_b$, $R_c$ and $R_d$ are each independently selected from methyl, ethyl, n-propyl, isopropyl, n-butyl, sec-butyl, isobutyl or tert-butyl;
particularly preferably, the organic silicate is selected from tetramethyl silicate, tetraethyl silicate, tetrabutyl silicate, dimethyl diethyl silicate, or a combination thereof.

**10.** The method according to any one of claims 7 to 9, wherein the first templating agent and the second templating agent are each independently selected from at least one of the quaternary ammonium base having a structure represented by the following formula (B):

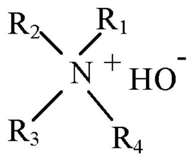
(B);

wherein $R_1$, $R_2$, $R_3$ and $R_4$ are each independently selected from alkyl group having 1 to 4 carbon atoms, preferably selected from linear alkyl group having 1 to 4 carbon atoms or branched alkyl group having 3 to 4 carbon atoms, and further preferably $R_1$, $R_2$, $R_3$ and $R_4$ are each independently selected from methyl, ethyl, n-propyl, isopropyl, n-butyl, sec-butyl, isobutyl and tert-butyl; or
the first templating agent and the second templating agent are each independently selected from tetraethylammonium hydroxide, tetrapropylammonium hydroxide, tetrabutylammonium hydroxide, tetrapropylammonium chloride, tetrapropylammonium bromide, or a combination thereof.

**11.** The method according to any one of claims 7 to 10, wherein in step 1), the titanium source is selected from one or more of an organic titanium source and an inorganic titanium source;

the organic titanium source is selected from at least one of the titanium-containing organic acid esters having the structure shown in the following formula (C):

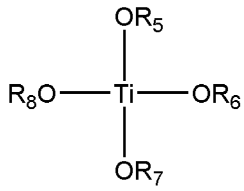

(C);

wherein $R_5$, $R_6$, $R_7$ and $R_8$ are each independently selected from alkyl group having 1 to 6 carbon atoms, preferably selected from linear alkyl group having 1 to 4 carbon atoms or branched alkyl group having 3 to 6 carbon atoms, and further preferably $R_5$, $R_6$, $R_7$ and $R_8$ are each independently selected from linear alkyl group having 2 to 4 carbon atoms or branched alkyl group having 3 to 4 carbon atoms; optionally, $R_5$, $R_6$, $R_7$ and $R_8$ are each independently selected from methyl, ethyl, n-propyl, isopropyl, n-butyl, sec-butyl, isobutyl, tert-butyl, isopentyl, hexyl or isohexyl; preferably, each independently selected from ethyl, n-propyl, isopropyl, n-butyl, sec-butyl, isobutyl or tert-butyl;

the inorganic titanium source is selected from one or more of chlorides, nitrates and sulfates of titanium;

preferably, the titanium source is selected from titanium tetrachloride, titanium sulfate, titanium nitrate, tetraethyl titanate, tetrapropyl titanate and tetrabutyl titanate, or a combination thereof.

**12.** The method according to any one of claims 7 to 11, wherein in step 1):

the carbon material used as the hard templating agent is selected from carbon nanoparticles, carbon nanotubes, carbon nanofibers and ordered mesoporous carbon, or a combination thereof;

the resin material used as the hard templating agent is selected from phenolic resin, polyester resin and polyamide resin, or a combination thereof;

the solid inorganic compound used as the hard templating agent is selected from calcium carbonate, magnesium hydroxide and sodium carbonate, or a combination thereof.

**13.** The method according to any one of claims 7 to 12, wherein in step 1), the high molecular weight polymer is selected from polyethylene, polypropylene, polyvinyl chloride, polystyrene, polyvinyl alcohol, polyvinyl acetate, polyacrylonitrile, polymethyl methacrylate, polyvinyl butyral, polyethyleneimine, 4-polyvinyl pyridine and poly(diallyl dimethyl ammonium chloride), or a combination thereof;

preferably, the high molecular weight polymer is selected from polyethylene, polypropylene, polystyrene and polyacrylonitrile, or a combination thereof;

optionally, the weight average molecular weight of the high molecular weight polymer is 10,000-200,000.

**14.** The method according to any one of claims 7 to 13, wherein step 1) comprises:

a) mixing the titanium source, the silicon source, the first templating agent and water to obtain a hydrolyzed sol containing titanium and silicon;

b) adding the hard templating agent and the high molecular weight polymer to the hydrolyzed sol containing titanium and silicon respectively, and mixing them to obtain the reaction mixture;

optionally, the mixing conditions in step a) include: stirring at 40-90°C for 6-12 h;

optionally, the mixing conditions in step b) include: stirring at 20-50°C for 2-4 h;

preferably, the silicon source is an organic silicate, and step a) further comprises a hydrolysis and alcohol removal treatment after mixing the titanium source, the silicon source, the first templating agent and water to obtain the hydrolyzed sol containing titanium and silicon;

optionally, the conditions of the hydrolysis and alcohol removal treatment include: stirring and hydrolyzing at 40-90 °C for 6-12 h; preferably stirring and hydrolyzing at 60-85 °C for 8-10 h.

**15.** The method according to any one of claims 7 to 14, wherein the conditions of the first hydrothermal crystallization treatment in step 2) and the second hydrothermal crystallization treatment in step 3) independently include: a hydrothermal crystallization temperature of 120-220°C, preferably 140-185°C; a hydrothermal crystallization time of 4-180h, preferably 6-80h; a pressure of autogenous pressure; and/or

the conditions of the first calcination treatment in step 2) and the second calcination treatment in step 3) independently include: a calcination temperature of 350-750° C, preferably 380-620° C; and a calcination time of 1-15 h, preferably

1.5-4.5 h.

16. The method according to any one of claims 7 to 15, wherein in step 3), the weight ratio of the second templating agent: water: the molecular sieve intermediate is (0.08-3.5): (0.5-25): 1; preferably (0.12-2.5): (2-18): 1.

17. A method for preparing caprolactam from cyclohexanone oxime, comprising: a step of subjecting cyclohexanone oxime to a gas-phase Beckmann rearrangement reaction in the presence of the titanium silicalite molecular sieve with a hierarchical porous structure according to any one of claims 1 to 6.

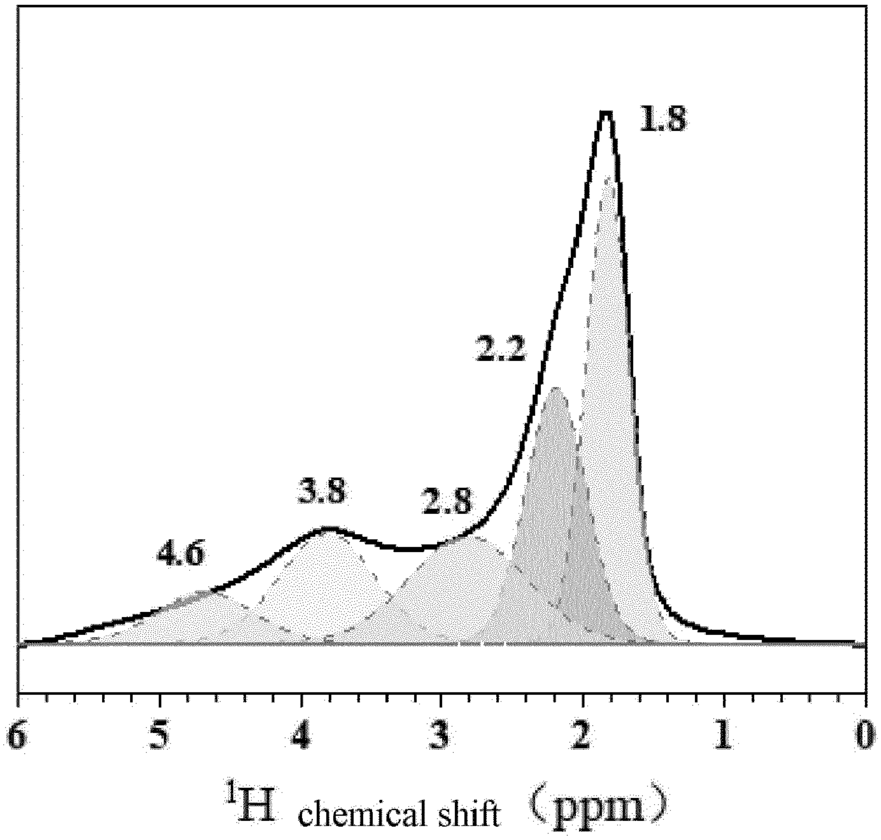
1.8
2.2
3.8
2.8
4.6
6
5
4
3
2
1
0
$^1$H chemical shift (ppm)

Fig. 1

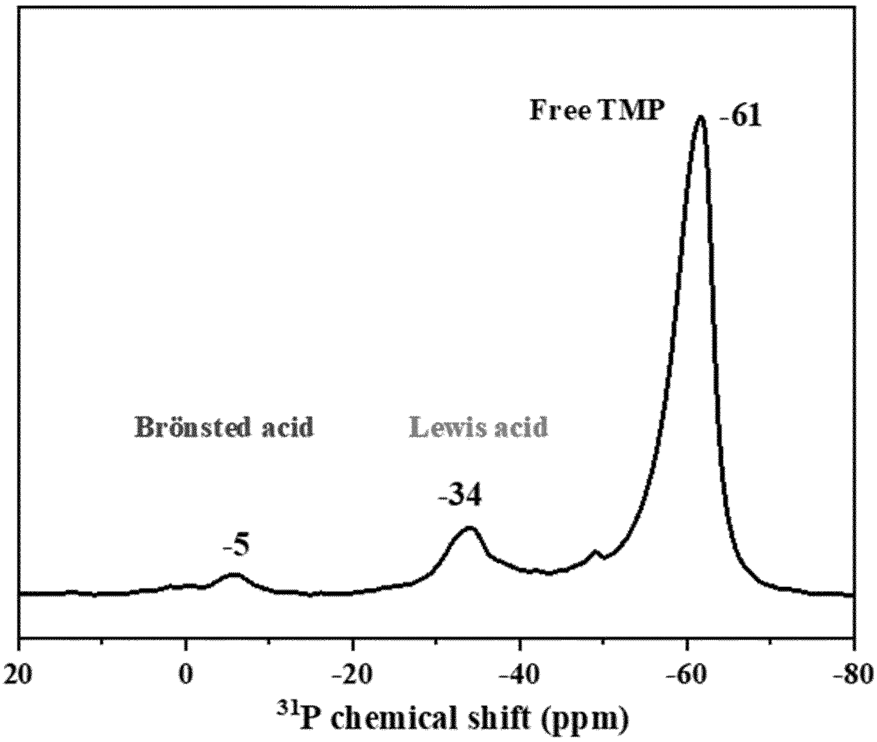
Free TMP
-61
Brönsted acid
Lewis acid
-34
-5
20
0
-20
-40
-60
-80
$^{31}$P chemical shift (ppm)

Fig. 2

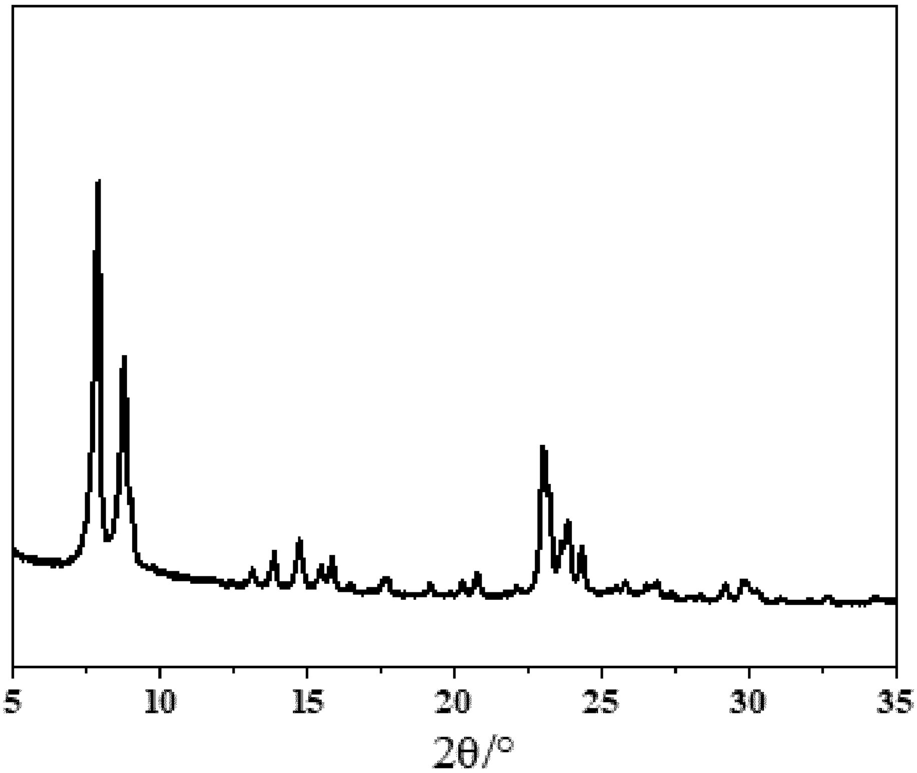


Fig. 3

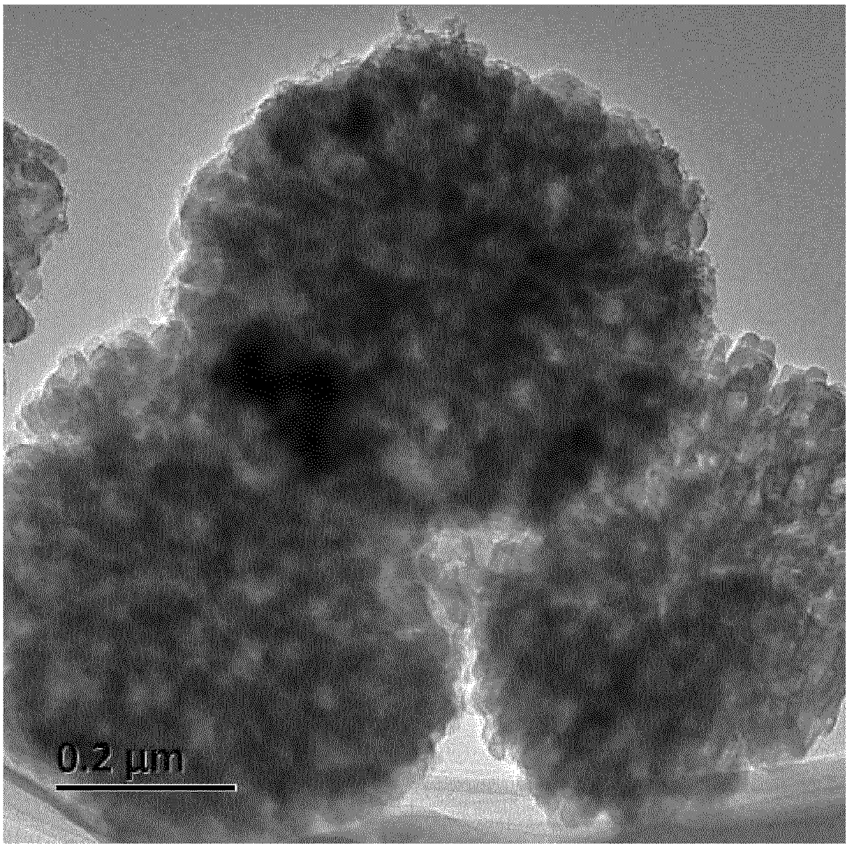


Fig. 4

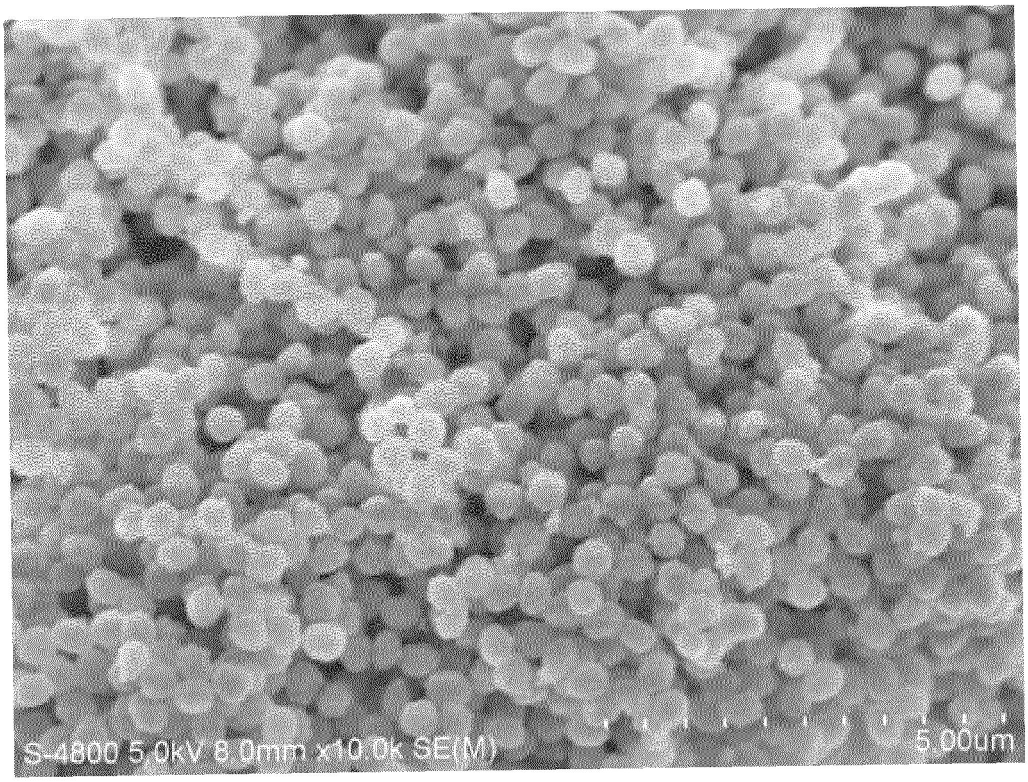
S-4800 5.0kV 8.0mm x10.0k SE(M)
5.00um

Fig. 5

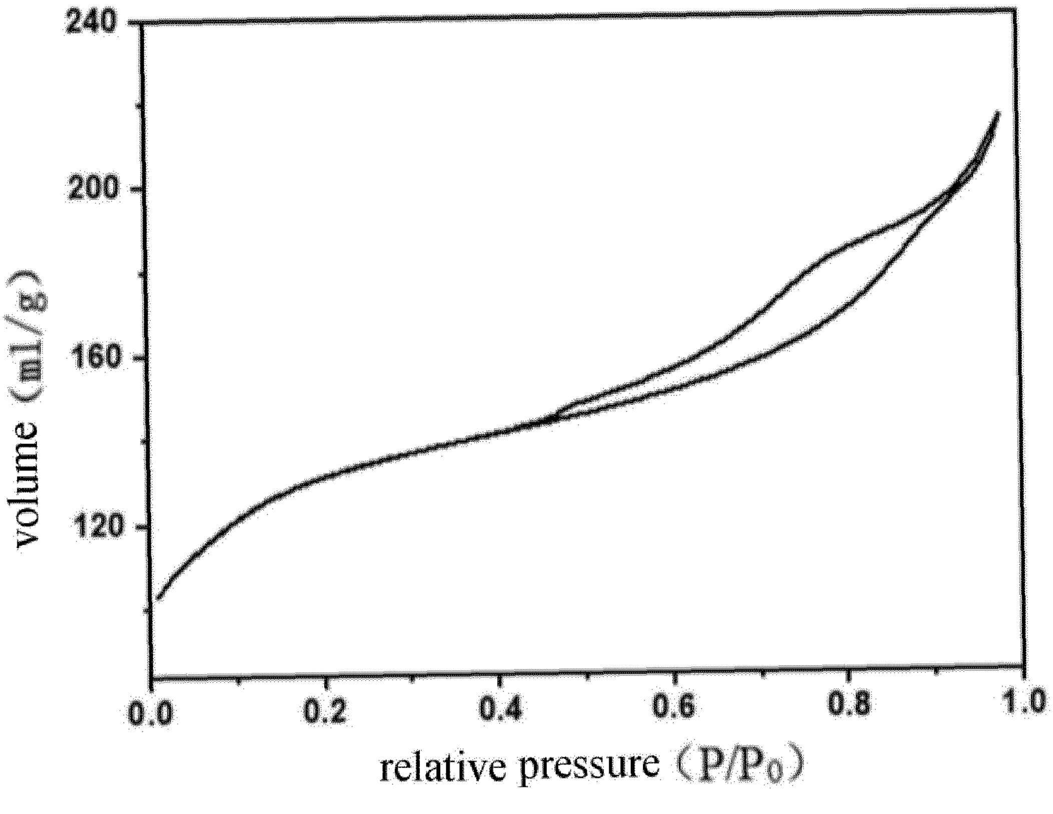
240
200
160
120
volume (ml/g)
0.0
0.2
0.4
0.6
0.8
1.0
relative pressure (P/P0)

Fig. 6

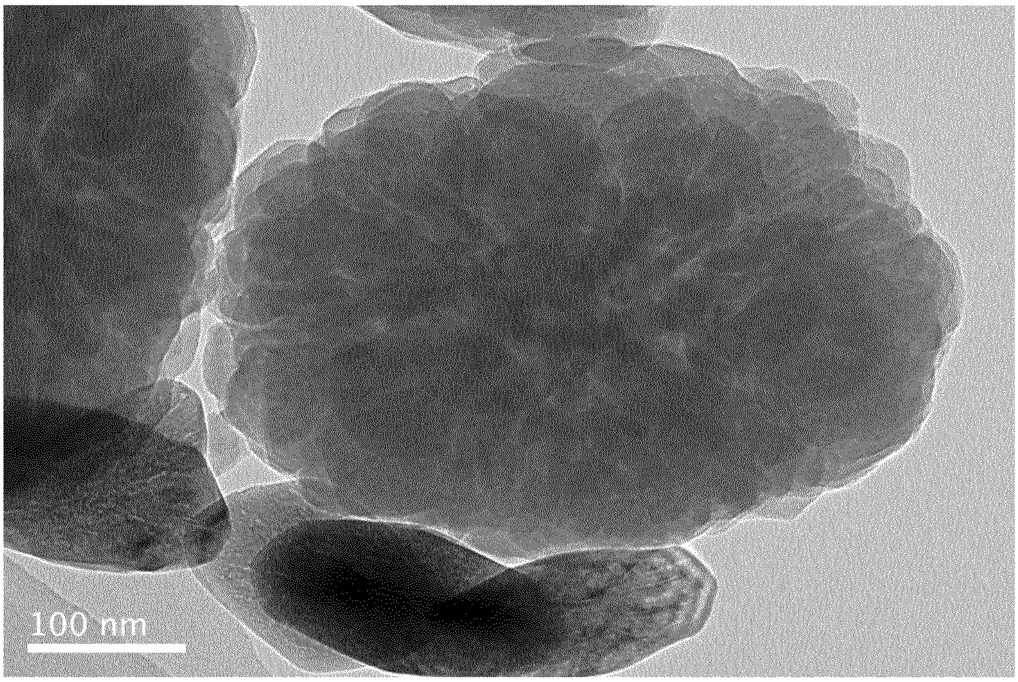


Fig. 7

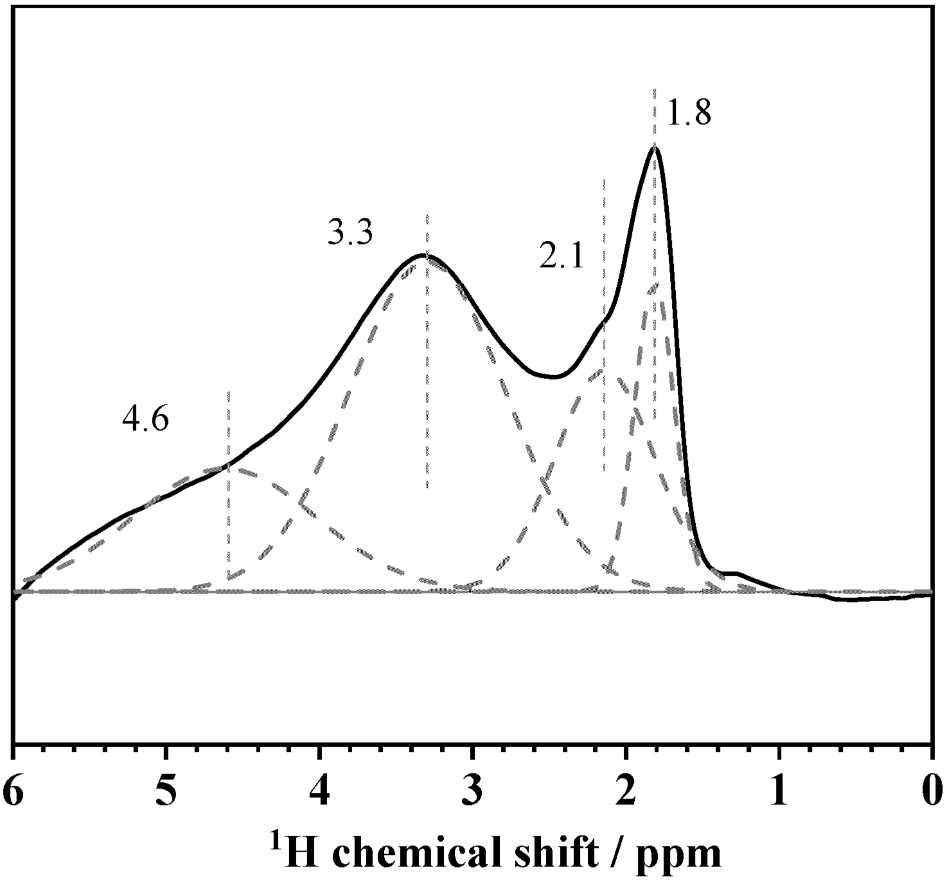


Fig. 8

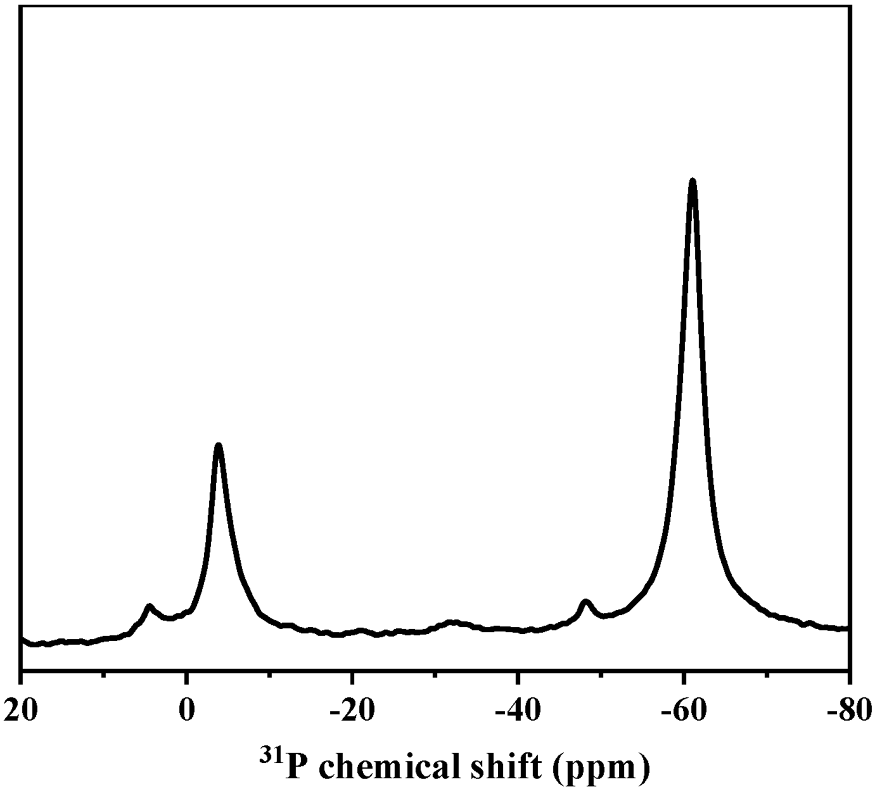


Fig. 9

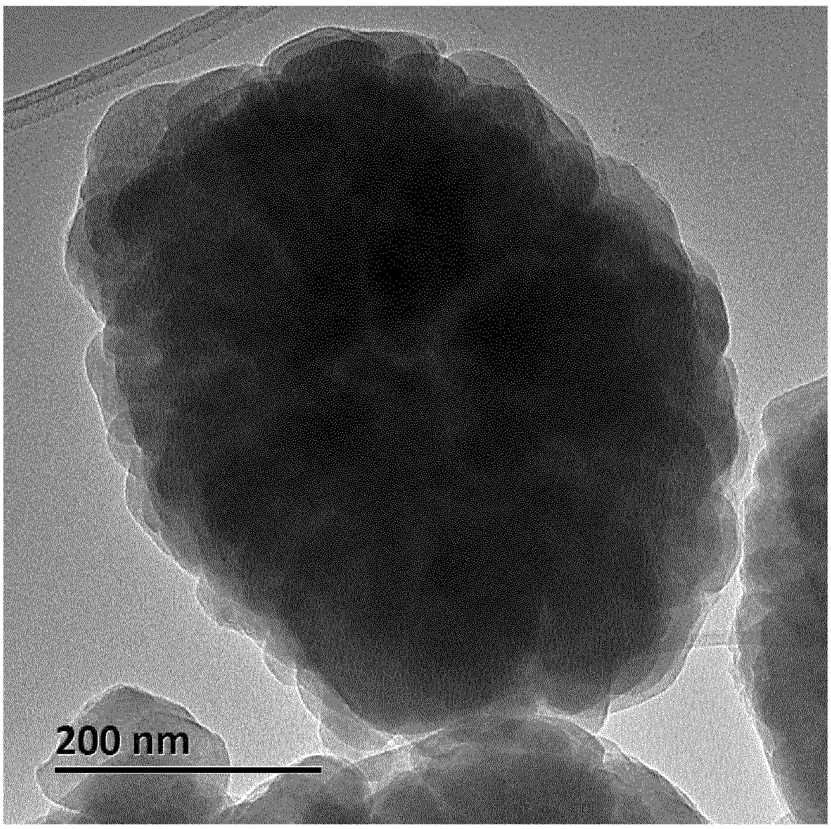


Fig. 10

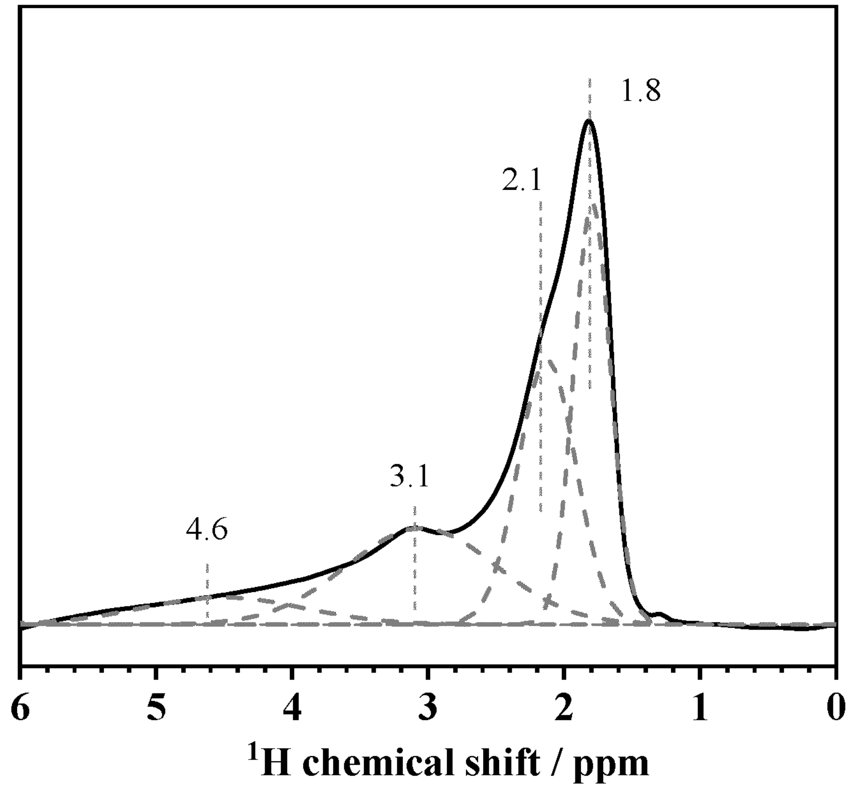
1.8
2.1
3.1
4.6
6
5
4
3
2
1
0
$^1$H chemical shift / ppm

Fig. 11

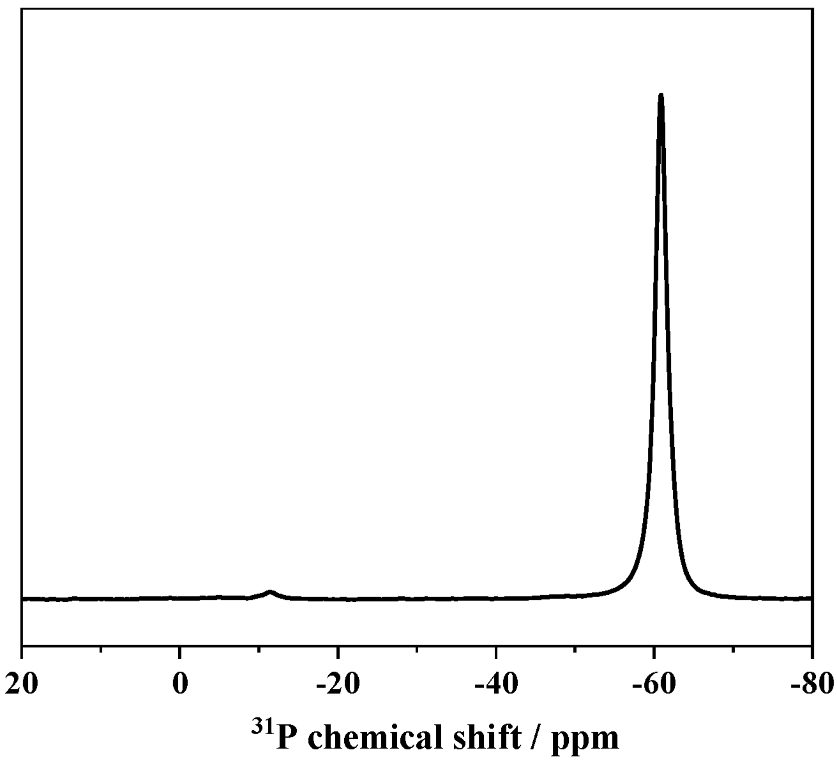
20
0
-20
-40
-60
-80
$^{31}$P chemical shift / ppm

Fig. 12

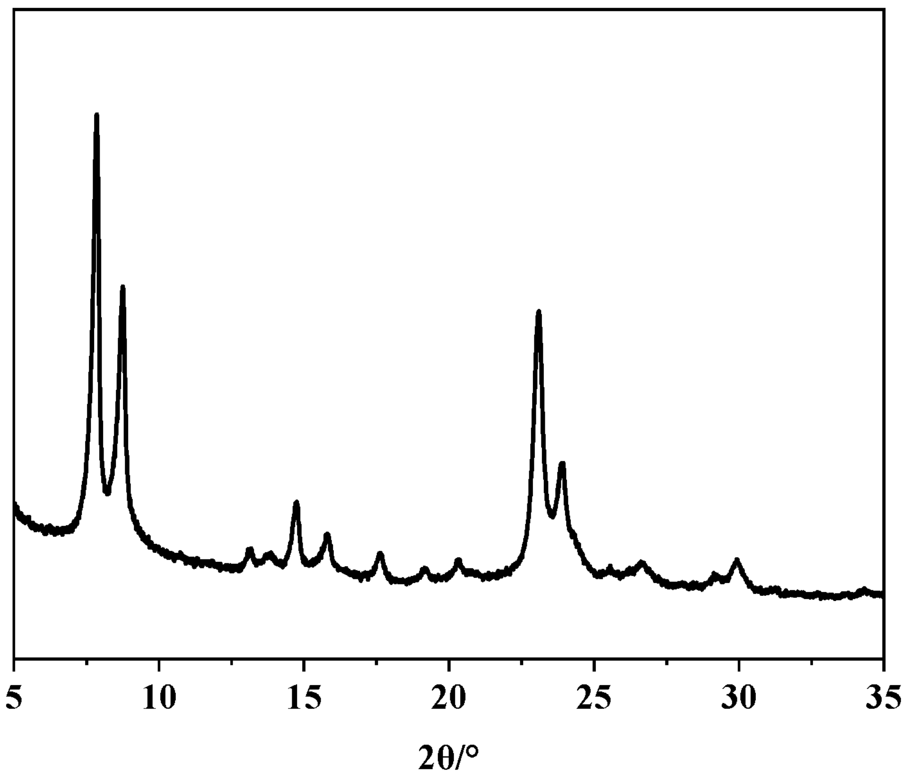


Fig. 13

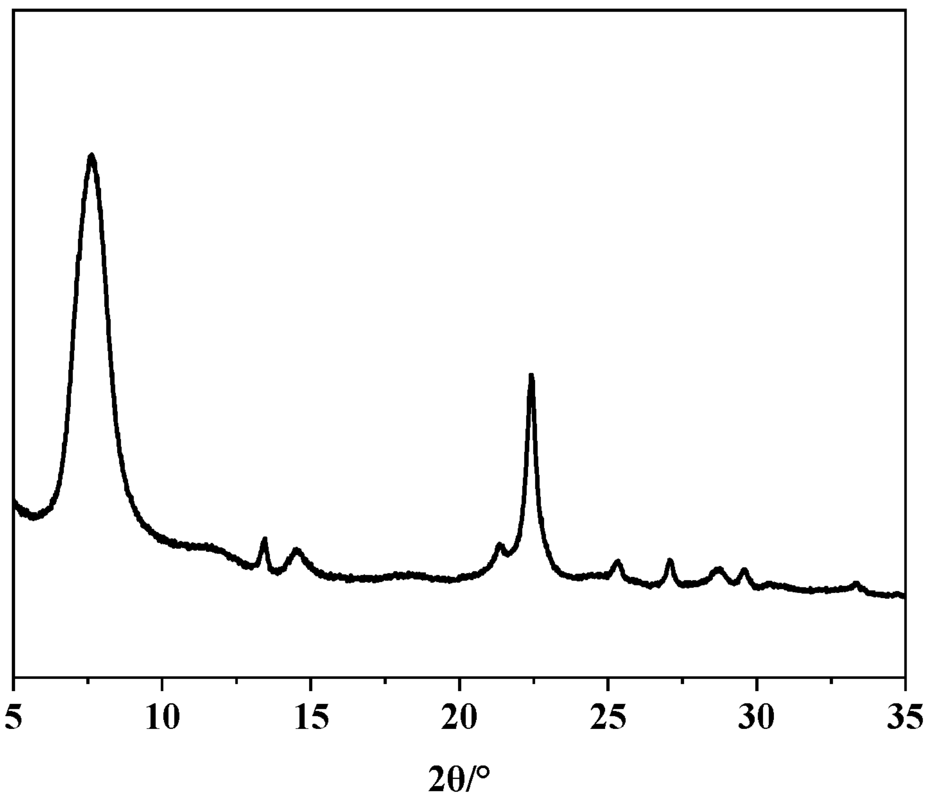


Fig. 14

**INTERNATIONAL SEARCH REPORT**

International application No. **PCT/CN2023/127042**

**A. CLASSIFICATION OF SUBJECT MATTER**

C01B 39/08(2006.01)i； B01J29/89(2006.01)i； C07C249/04(2006.01)i

According to International Patent Classification (IPC) or to both national classification and IPC

**B. FIELDS SEARCHED**

Minimum documentation searched (classification system followed by classification symbols)

IPC：C01B；B01J；C07C

Documentation searched other than minimum documentation to the extent that such documents are included in the fields searched

Electronic data base consulted during the international search (name of data base and, where practicable, search terms used)

CNTXT, DWPI, WPABS, ENTXTC, 中国期刊网全文数据库 China Journal Network Full-text Database (CJFD), ISI Web of Science: 中国石油化工股份有限公司, 中石化石油化工科学研究院有限公司, 夏长久, 张鹏, 彭欣欣, 邢恩会, 钛 2d 硅, TS, 多级孔, 分级, 介孔, 微孔, 大孔, 分子筛, 沸石, NMR, 核磁, 硅羟基, 氢键, 碳纳米, 介孔碳, 介孔炭, 树脂, 碳酸钙, 氢氧化镁, 碳酸钠, CaCO3, Na2CO3, 高分子, 聚合物, 聚 2d 烯, 模板, 催化, 环己酮肟, titanium silicate, titanosilicate, hierarchical+, mesoporous, microporous, macroporous, carbon nano+, carbon black, mesoporous carbon, resin, calcium carbonate, magnesium hydroxide, sodium carbonate, polymer, zeolite?, molecular sieve?, templat+, cataly+, cyclohexanone

**C. DOCUMENTS CONSIDERED TO BE RELEVANT**

| Category* | Citation of document, with indication, where appropriate, of the relevant passages | Relevant to claim No. |
|---|---|---|
| A | CN 106145151 A (CHINA PETROLEUM & CHEMICAL CORP. et al.) 23 November 2016 (2016-11-23) description, paragraphs 30-37 | 1-17 |
| A | CN 106276958 A (WUHAN UNIVERSITY OF TECHNOLOGY) 04 January 2017 (2017-01-04) entire document | 1-17 |
| A | CN 107915234 A (EAST CHINA NORMAL UNIVERSITY) 17 April 2018 (2018-04-17) entire document | 1-17 |
| A | CN 112138709 A (ZHEJIANG HENGLAN TECHNOLOGY CO., LTD.) 29 December 2020 (2020-12-29) entire document | 1-17 |

☑ Further documents are listed in the continuation of Box C. ☑ See patent family annex.

* Special categories of cited documents:
- “A” document defining the general state of the art which is not considered to be of particular relevance
- “D” document cited by the applicant in the international application
- “E” earlier application or patent but published on or after the international filing date
- “L” document which may throw doubts on priority claim(s) or which is cited to establish the publication date of another citation or other special reason (as specified)
- “O” document referring to an oral disclosure, use, exhibition or other means
- “P” document published prior to the international filing date but later than the priority date claimed
- “T” later document published after the international filing date or priority date and not in conflict with the application but cited to understand the principle or theory underlying the invention
- “X” document of particular relevance; the claimed invention cannot be considered novel or cannot be considered to involve an inventive step when the document is taken alone
- “Y” document of particular relevance; the claimed invention cannot be considered to involve an inventive step when the document is combined with one or more other such documents, such combination being obvious to a person skilled in the art
- “&” document member of the same patent family

| Date of the actual completion of the international search | Date of mailing of the international search report |
|---|---|
| **07 December 2023** | **18 December 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/127042**

**C. DOCUMENTS CONSIDERED TO BE RELEVANT**

| Category* | Citation of document, with indication, where appropriate, of the relevant passages | Relevant to claim No. |
|---|---|---|
| A | CN 112744836 A (CHINA PETROLEUM & CHEMICAL CORP. et al.) 04 May 2021 (2021-05-04)<br>entire document | 1-17 |
| A | KR 20110121474 A (INHA-INDUSTRY PARTNERSHIP INSTITUTE) 07 November 2011 (2011-11-07)<br>entire document | 1-17 |
| A | 夏长久等 (XIA, Changjiu et al.). "多尺度孔道分布钛硅分子筛材料制备策略的探讨 (Synthesis Strategies for Titanium Silicate Zeolites With Multimodal Porous Networks)" *石油学报 (石油加工) (Acta Petrolei Sinica (Petroleum Processing Section)),* Vol. 32, No. 2, 30 April 2016 (2016-04-30), 407-417<br>ISSN: 1001-8719,<br>entire document | 1-17 |

**INTERNATIONAL SEARCH REPORT**
**Information on patent family members**

International application No.
**PCT/CN2023/127042**

| Patent document cited in search report | | | Publication date (day/month/year) | Patent family member(s) | | | Publication date (day/month/year) |
|---|---|---|---|---|---|---|---|
| CN | 106145151 | A | 23 November 2016 | CN | 106145151 | B | 30 November 2018 |
| CN | 106276958 | A | 04 January 2017 | CN | 106276958 | B | 25 January 2019 |
| CN | 107915234 | A | 17 April 2018 | CN | 107915234 | B | 16 October 2020 |
| CN | 112138709 | A | 29 December 2020 | | None | | |
| CN | 112744836 | A | 04 May 2021 | CN | 112744836 | B | 28 June 2022 |
| KR | 20110121474 | A | 07 November 2011 | | None | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 195872004 T
- CN 112744836 A


### Non-patent literature cited in the description


- **BAOJUN LI**. *RSC Adv*, 2013, vol. 3, 20811-20815
- **FERDI SCHÜTH et al.** *Microporous and Mesoporous Materials*, 2009, vol. 117, 228-232